# EUROPEAN PATENT SPECIFICATION

(11) **EP 3 571 197 B1**
(45) Date of publication and mention of the grant of the patent: **14.10.2020**
(21) Application number: 18700378.5
(22) Date of filing: 17.01.2018
(51) Int. Cl.: C07D 471/04, C09K 11/06, H01L 31/055, H01L 33/50, C09B 57/08, C09B 67/22

(54) **FLUORESCENT COLORANTS BASED ON CYANOARYL-SUBSTITUTED NAPHTHOYLENEBENZIMIDAZOLE COMPOUNDS**
FLUORESZIERENDE FARBSTOFFE AUF DER BASIS VON CYANOARYL-SUBSTITUIERTEN NAPHTHOYLENBENZIMIDAZOLVERBINDUNGEN
COLORANTS FLUORESCENTS À BASE DE COMPOSÉS DE NAPHTOYLÈNE BENZIMIDAZOLE À SUBSTITUTION CYANOARYLE

(30) Priority: 18.01.2017 EP 17151931
(43) Date of publication of application: 27.11.2019
(73) Proprietor: BASF SE, 67056 Ludwigshafen am Rhein (DE)
(72) Inventor: KOENEMANN, Martin, 67056 Ludwigshafen (DE); WAGENBLAST, Gerhard, 67157 Wachenheim (DE); IVANOVICI, Sorin, 67056 Ludwigshafen (DE)
(74) Representative: BASF IP Association
(86) International application number: PCT/EP2018/051119
(87) International publication number: WO 2018/134261

(56) References cited:
- WO-A1-2012/168395
- WO-A1-2015/019270

## Description

### FIELD OF THE INVENTION

The present invention relates to cyanoaryl-substituted naphthoylenebenzimidazole compounds which are useful as fluorescent colorants and to their use in color converters for converting light emitted from a blue LED into light of a second, longer wavelength or for converting light emitted from a white LED to provide white light having a lower correlated color temperature. The present invention also relates to these color converters, to their use in lighting devices, to lighting devices comprising at least one LED and at least this color converter and to a device producing electric power upon illumination comprising a photovoltaic cell and said color converter.

### BACKGROUND OF INVENTION

Incandescent lamps and halogen lamps are commonly used as light sources. Being thermal radiators, they produce light with very good color reproduction, since a broad spectrum is emitted with radiation characteristics approaching Planck's law of black body radiation and closely resembling sunlight. A disadvantage of incandescent lamps and fluorescent lamps is the high power consumption, since a very large amount of electrical energy is converted to heat and a significant part of the emitted light is out of the visible spectral range.

Light emitting diodes (LEDs) are replacing conventional light sources such as incandescent lamps and fluorescent lamps for general lighting applications due to their much higher energy efficiency and longer lifetime. The light emission is based on the recombination of electron-hole pairs (excitons) in the junction area of a forward-biased semiconductor pn junction. The size of the band gap of this semiconductor determines the approximate wavelength of the emitted light. LEDs generate light in a narrow spectral wavelength range, where the center wavelength of emission is determined by the semiconductor materials forming the active layer. For examples blue to green LEDs can be produced by using nitride semiconductors such as AIN (aluminium nitride), GaN (gallium nitride), InN (indium nitrirde) or InGaN (indium gallium nitride).

White light illumination is often required for general lighting purpose as well as in a wide range of other applications such as backlights for displays. So far, there exists no LED which can emit white light directly. In order to create white light, a lighting device comprising an LED, preferably a blue LED, and a wavelength converting material also referred to as phosphor, is often used.

White-light LEDs can be created from blue- or near-ultraviolet-emitting LED chips coated or covered with a yellow luminescent material. According to this concept, the luminescent material is applied directly and without intervening space to the LED light source (LED chip). This concept is also referred to as "phosphor on a chip". In phosphor on a chip LEDs, the luminescent material used is generally an inorganic material. The luminescent material may be a single phosphor material or a mixture of different phosphor materials. The luminescent material, which may consist, for example, of cerium-doped yttrium aluminium (Ce:YAG) garnet, absorbs a certain proportion of the blue light and emit longer-wave light with a broad emission band, such that the mixing of the transmitted blue light and of the emitted light gives rise to white light. Nowadays, the most widely used white light-emitting diodes are phosphor-converted LEDs made of the InGaN blue-emitting chip and cerium-doped yttrium aluminium garnet as yellow phosphor. White-light LEDs of this type have a correlated color temperature CCT of greater than 6 000 K, i.e., they generate cool white light, since the red component in the spectrum of the generated white light is too weak. In addition, their average color rendering index CRI is low, usely about 70 to 85.

To provide a more pleasing and natural white light having a CCT of below 6 000 K, a different concept may be used. According to this concept, the luminescent material is dissolved or dispersed in a polymeric matrix which is at a certain distance from the blue LED chip. This structure is referred to as "remote phosphor". The spatial distance reduces the stress resulting from heat and radiation to such an extent that the requirements on the stability can be achieved by suitable organic phosphors. The luminescent material may be a single phosphor material. Often, a mixture of different phosphors is used to broaden the spectrum, for example a mixture of yellow and red fluorescent organic colorants.

White light LEDs having a CCT below 6 000 K may also be constructed from a phosphor-on a chip LED made of a blue-emitting LED chip and an inorganic yellow phosphor such as cerium-doped yttrium aluminium garnet in combination with a color converter comprising at least one organic phosphor, where the phosphor-on a chip LED and the color converter are in a spatial distance (remote phosphor arrangement). Often, the color converter comprises a combination of a yellow organic phosphor or a green organic phosphor and a red organic phosphor.

Color reproduction and color temperature are important characteristics of LED lightings. Color reproduction also referred to as "Color Rendering" describes the ability to accurately render all colors of the spectrum. The average color rendering index (CRI Ra) represents the color rendering properties of selected eight pastel test colors. The CRI Ra does not take into consideration saturated colors, for example saturated red. Sample color R9 is the saturated red color. Often, the ability to reproduce red colors well is essential for accurately rendering colors, as the color red is often found mixed into processed colors. Accordingly, light sources with a high R9 color rendering value are desirable to reproduce reddish colors vividly. Color temperature describes the color tint of the white light. It is referred to as "Correlated Color Temperature" (CCT) measured in Kelvin, since LEDs are not incandescent radiators.

The selection of the luminescent conversion material is critical for the targeted color temperature and the color reproduction of the lighting device. Sunlight has output at all visible wavelengths with relatively gradual and smooth transitions in contrast to known commercial white LEDs. Commercial white LEDs such as a white LED based on a blue LED coated with yellow phosphor such as Ce:YAG are ordinarily used because of its broad emission spectrum. The spectrum is rich in a blue component and in a broad yellow component, but the red and green components of the white light are relatively weak which in turn results an incomplete coverage of the full range of visible wavelengths. The green gap in spectral energy of white LEDs and the relative lack in wavelengths other than those emitted by the blue LED limit the color rendering. A color range, which is impaired to a great extend by such white LED light, is the green spectral range. For example, when an object is illuminated under the known white LEDs, they cannot well imitate the colors illuminated by natural light. Thus, there is a need for organic phosphors that efficiently absorb excitation energy from the blue LED and emit at wavelengths longer than those of the blue-emitting LED but shorter than those of the yellow inorganic phosphor emission to improve the color rendering in white light emitting lighting devices.

For general lighting applications, backlighting and various other applications, good color reproduction (color rendering) (e.g. a CRI Ra of greater than 90) is often essential. Especially, there is a need for lighting devices generating white light having (i) a warm-white or neutral-white color, corresponding to a correlated color temperature CCT of less than 6 000 K, especially less than 5 000 K, more especially less than 4 000 K or even less than 3 000 K and (ii) having a good color reproduction corresponding to a CRI Ra of greater than 90, preferably greater than 92 and especially greater than 95.

Numerous organic fluorescent compounds have been proposed in the prior art which can be excited by a blue LED and re-emit in the wavelength range from 490 - 600 nm.

WO 2012/168395 describes color converters comprising at least one polymer and at least one organic yellow-fluorescent colorant, wherein the organic fluorescent colorant comprises at least one structural unit of the formula (A), 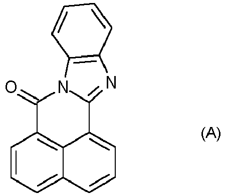 where the structural unit may be mono- or polysubstituted by identical or different substituents and where one or more CH groups in the six-membered ring of the benzimidazole structure shown may be replaced by nitrogen. This reference also describes color converters comprising at least one phosphor comprising at least one structural unit of formula (A) in combination with a red organic phosphor.

WO 2015/019270 describes cyanated naphthoylenebenzimidazole compounds and their use as yellow-fluorescent colorant, in particular their use in color converters. The reference also describes white LEDs using a blue LED to pump a mixture comprising at least one cyanated naphthoylenebenzimidazole compound in combination with a red organic phosphor. Said cyanated naphthoylenebenzimidazole compounds often suffer from multistage syntheses andr the process also often requires an elaborate procedure for the purification.

The above-mentioned compounds of prior art are often not able to close the gap in the green spectral range typically seen in white LED light.

It is therefore an object of the present invention to provide organic fluorescent colorants which can absorb excitation energy from blue LEDs and emit in the green-yellow wavelength range (wavelength range between 490 and 540 nm. The novel organic phosphors should also be suitable for use in color converters for obtaining white light having a desired CCT and a high average color rendering index CRI Ra and/or R9 value. The novel fluorescent compounds should especially be suitable in combination with further colorants for creating white light having a CCT below 6 000 K with high color quality (CRI Ra >90) and positive R9 value (especially R9 > 66).

In addition, there is an ongoing need for organic fluorescent colorants which have one or more of the following characteristics:
- high light stability under blue light irradiation conditions;
- high heat stability under blue light irradiation conditions;
- high chemical stability with respect to moisture and oxygen;
- long lifetime;
- high fluorescence quantum yield; and
- easily obtainable in pure form.

Alternatively or additionally, it is an object of the present invention to provide a lighting device having a CCT below 6 000 K, whose average color rendering index CRI is greater than 90, preferably at least 92 and in particular more than 95. It is also an object of the present invention to provide a lighting device having a CCT below 6 000 K and a R9 vlaue greater than 66.

New naphthoylenebenzimidazole compounds have now been found which are advantageously suitable for creating white light with high color rendering index CRI Ra.

### SUMMARY OF THE INVENTION

The present invention is thus based on the object of providing organic fluorescent compounds or mixture of organic fluorescent compounds, especially for use in color converters.

Hence, in a first aspect, the present invention relates to a naphthoylenebenzimidazole compound of the formula (I) 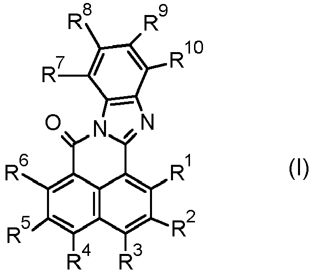 wherein
at least one of the radicals R¹, R², R³, R⁴, R⁵, R⁶, R⁷, R⁸, R⁹ and R¹⁰, independently of each other is aryl which carries one, two or three cyano groups and 0, 1, 2, 3 or 4 identical or different substituents R^{Ar} and the remaining radicals R¹, R², R³, R⁴, R⁵, R⁶, R⁷, R⁸, R⁹ and R¹⁰ independently of each other are selected from hydrogen and aryl which is unsubstituted or carries 1, 2, 3, 4 or 5 substituents R^{Ar},
and wherein at least one of the radicals R⁷, R⁸, R⁹ and R¹⁰ is aryl which carries 1, 2 or 3 cyano groups and 0, 1, 2, 3 or 4 substituents R^{Ar} and the remaining radicals R⁷, R⁸, R⁹ and R¹⁰ are, independently of each other, selected from the group consisting of hydrogen and aryl which is unsubstituted or carries 1, 2 or 3 radicals R^{Ar};
where
- R^{Ar}: independently of each other and independently of each occurrence, is selected from halogen,
C₁-C₃₀-alkyl, C₂-C₃₀-alkenyl, C₂-C₃₀-alkynyl, where the three latter radicals are unsubstituted or carry one or more R^{a} groups, C₃-C₈-cycloalkyl, 3- to 8-membered heterocyclyl, where the two latter radicals are unsubstituted or carry one or more R^{b} groups, aryl and heteroaryl, where the two latter radicals are unsubstituted or carry one or more R^{c} groups, where
R^{a} ,independently of each other and independently of each occurrence, is selected from cyano, halogen, C₃-C₈-cycloalkyl, 3- to 8-membered heterocyclyl, aryl and heteroaryl, where C₃-C₈-cycloalkyl, 3- to 8-membered heterocyclyl are unsubstituted or bear one or more R^{b1} groups, and where aryl and heteroaryl are unsubstituted or bear one or more R^{c1} groups;
R^{b} ,independently of each other and independently of each occurrence, is selected from cyano, halogen, C₁-C₁₈-alkyl, C₃-C₈-cycloalkyl, 3- to 8-membered heterocyclyl, aryl and heteroaryl, where C₃-C₈-cycloalkyl, 3- to 8-membered heterocyclyl are unsubstituted or bear one or more R^{b1} groups, and where aryl and heteroaryl are unsubstituted or bear one or more R^{c1} groups;
R^{c} ,independently of each other and independently of each occurrence, is selected from cyano, halogen, C₁-C₁₈-alkyl, C₃-C₈-cycloalkyl, 3- to 8-membered heterocyclyl, aryl and heteroaryl, where C₃-C₈-cycloalkyl, 3- to 8-membered heterocyclyl are unsubstituted or bear one or more R^{b1} groups, and where aryl and heteroaryl are unsubstituted or bear one or more R^{c1} groups;
R^{b1} ,independently of each other and independently of each occurrence, is selected from halogen, C₁-C₁₈-alkyl and C₁-C₁₈-haloalkyl,
R^{c1} ,independently of each other and independently of each occurrence, is selected from halogen, C₁-C₁₈-alkyl and C₁-C₁₈-haloalkyl;
or a mixture thereof.

The novel compounds of formula (I) are very advantageous with regard to their use in color converters, since they emit in the green wavelength range thus closing the gap in the green spectral range. In addition, the novel compounds have the advantage that they can easily be prepared and they are obtained in high purity. A color converter comprising a compound of formula (I) and further organic fluorescent colorants being different from compounds of formula (I) are suitable for obtaining white light having an average color rendering index CRI Ra of greater than 90 and a R9 value of greater than 66.

Thus, in a further aspect, the present invention relates to the use of a compound of the formula (I) as defined above or a mixture thereof in color converters for converting light emitted from a white LED having a correlated color temperature between 3 000 K and 20 000 K to provide white light having a lower correlated color temperature.

In a further aspect, the present invention relates to the use of compound of the formula (I) as defined above or a mixture thereof in color converters for converting light emitted from a blue LED with a center wavelength of emission between 400 nm and 480 nm into light of a second, longer wavelength to provide white light having an average color rendering index CRI Ra of greater than 90 and a R9 value of greater than 66.

The color converters according to the present invention allow to generate white light with a targeted correlated color temperature e.g. below 6 000 K, especially below 5 000 K, more especially below 4 000 K or below 3 000 K which has a high average color rendering index (CRI Ra > 90) and high R9 value (R9 > 66).

In yet a further aspect, the present invention relates to a color converter comprising in a polymeric matrix at least one naphthoylenebenzimidazole compound of the formula (I) as defined above or a mixture thereof as fluorescent colorant and at least one further organic fluorescent colorant which is selected from
(a) an organic orange fluorescent colorant having a maximum of emission in the wavelength range of 540 to less than 600 nm and mixtures thereof; and
(b) an organic red fluorescent colorant having a maximum of emission in the wavelength range of 600 to 670 nm and mixtures thereof;
and optionally at least one inorganic white pigment as scattering body..

In yet a further aspect, the present invention relates to the use of the color converter as defined above for conversion of light generated by a blue LED with a center wavelength of emission between 400 to 480 nm, especially 420 nm and 480 nm, to provide white light or for conversion of light generated by a cool white LED having a correlated color temperature between 3 000 K and 20 000 K, espcially between 6 000 to 20 000 K, to provide white light having a lower correlated color temperature.

In yet a further aspect, the present invention relates to a lighting device comprising
(i) at least one LED selected from a blue LED with a center wavelength of emission from 400 to 480 nm, especially 420 nm to 480 nm, and a white LED having a correlated color temperature between 3 000 K and 20 000 K, especially between 6 000 K and 20 000 K; and
(ii) at least one color converter as defined above;
wherein the at least one color converter is in a remote arrangement from the at least one LED.

In yet a further aspect, the present invention relates to a device producing electric power upon illumination comprising a photovoltaic cell and the color converter as defined above, where at least a part of the light not absorbed by the photovoltaic cell is absorbed by the color converter.

### DETAILED DESCRIPTION OF THE INVENTION

Naphthoylenebenzimidazole compounds are also referred to as naphthalene benzimidazole compounds.

Fluorescent colorants include all materials which are capable of absorbing light of a particular wavelength and converting it to light of another wavelength. Organic fluorescent colorants may be organic fluorescent pigments or organic fluorescent dyes.

In the context of the present invention, the term "luminescent material(s)" is also referred to as phosphor(s). The luminescent materials may be inorganic solids or organic fluorescent colorants.

Accordingly, in the context of the present invention, the terms "phosphor" and "colorant" are used interchangeably to describe a luminescent material which converts light of a first wavelength to light of a second wavelength.

The term "conversion material" refers to a material that is excited by a photon of a first wavelength and emits photons of a second, different wavelength.

In the context of the present invention, "a phosphor-converted LED" refers to an LED element having a phosphor material layer coated thereon for converting or changing the color of the light emitted by the LED element to a different color.

A quantum dot is a nanocrystal made of semiconductor materials that is small enough to exhibit quantum mechanical properties. Quantum dots are showing remarkably narrow emission spectra, i.e. with extraordinary small FWHM (full width at half maximum). The color output of the dots can be tuned by controlling the size of the crystals. With a smaller size in quantum dots, the quantum dots emit light of a shorter wavelength.

In the context of the present invention, "color converter" is understood to mean all physical devices capable of absorbing light of particular wavelengths and converting it to light of a second wavelength. Color converters are, for example, part of lighting devices, especially those lighting devices which utilize LEDs or OLEDs as a light source, or of fluorescence conversion solar cells. Thus, the blue light may be (at least) partly converted into visible light of longer wavelengths than the excitation wavelengths.

In the context of the present invention, the term "center wavelength" of a given spectral distribution F(λ) is defined as the following average: λ_{c} = ∫λ•F(λ) dλ / ∫F(λ) dλ.

In the context of the present invention, a "blue LED" is understood to mean an LED which emits light in the blue range of the electromagnetic spectrum with a center wavelength of emission in the range of 400 to 480 nm, preferably 420 to 480 nm, more preferably 440 to 470 nm, most preferably at 440 to 460 nm. Suitable semiconductor materials are silicon carbide, zinc selenide and nitrides such as aluminum nitride (AIN), gallium nitride (GaN), indium nitride (InN) and indium gallium nitride (InGaN). LEDs typically have a narrow wavelength distribution that is tightly centered about their peak wavelength. Standard InGaN-based blue LEDs are fabricated on a sapphire substrate and peak emission wavelength is usually centered at 445 to 455 nm.

Light sources that are not incandescent radiators have correlated color temperatures. The correlated color temperature (CCT) is the temperature of a black body radiator that is perceived by the human eye to emit the same white light as the LEDs. The correlated color temperature (CCT) describes the color appearance of white light emitted from electric light sources and is measured in Kelvin. It is determined according to the CIE international standard. CCT from a white light source usually is in the range from 1 500 K to 20 000 K, especially 2 000 K to 20 000 K. White light having higher CCT contains relatively higher intensity in the short wavelength region (blue) and relatively lower intensity in the longer wavelength region (red) compared to white light with lower CCT. Accordingly, higher CCTs generally indicate white light having a more significant blue component or a cool tone while lower CCTs generally indicate light having a more significant red tint or a warm tone. A white light having a CCT in the range from 4 500 K to 20 000 K is often referred to as cool white light, a white light having a CCT in the range from 2 700 K to 3 200 K is often referred to as warm-white light and a white light having a CCT in the range between 3 200 K to 4 500 K is often referred to as neutral white. Warmer color temperatures are especially suitable for living spaces.

Color rendering (CRI) is a measure how a light source makes the color of an object appear to the human eye and how well subtle variations in color shade are revealed. According to CIE 17.4, International Lighting Vocabulary, color rendering (CRI) is defined as "the effect of an illuminant on the color appearance of objects by conscious or unconscious comparison with the color appearance under a reference illuminant". The average or general color rendering index Ra is calculated from the differences in the chromaticities of the eight pastel CIE standard (reference) color samples R1 to R8 (CIE 13.3-1995). Negative values are also possible. A reference source, such as black body radiation, is defined as having a CRI index (Ra) of 100 (which is the maximum), i.e. a value of 100 indicates that the source renders colors in a manner identical to the reference. The lower the CRI rating, the less accurately colors will be reproduced. For many general interior illumination applications, a CRI value (Ra) of greater than 80 is acceptable. For general lighting, the color rendering index should be above 85. In applications where accurate color rendering is required, a high CRI Ra of at least 90 is usually highly desirable, so that objects illuminated by the lighting source may appear to have more natural coloring to the human eye.

CRI Ra does not include coefficients corresponding to six highly saturated colors (R9 - R14). Of these, R9 corresponds to a strong red color, which may affect a red-green contrast that may be beneficial in rendering colors. Often, the ability to reproduce red colors well is essential for accurately rendering colors, as the color red is often found mixed into processed colors. Thus, if a light source cannot render red correctly, things that are reddish will turn dull. Accordingly, light sources with high CRI Ra and with positive R9 value tend to produce the most vivid colors.

According to the CIE 1931 standard colorimetric system, colors are perceived by human eye following specific color curves. The standard luminosity curve Vλ accounts for the wavelength dependence of the sensitivity of human eye. The luminosity curve has a maximum possible value of 683 Im/W, for the case of monochromatic light at a wavelength of 555 nm (green). Luminous flux is the measure of the perceived power of light.

The term "essentially" in the context of the present invention encompasses the words "completely", "wholly" and "all". The word encompasses a proportion of 90% or more, such as 95% or more, especially 99% or 100%.

In the context of the invention, the phrase "compound of formula (I)" is also expressed as "compound of formula I", "compound (I)" or "compound I".

In the context of the invention, the phrase "aryl which is unsubstituted or carries 1, 2, 3, 4 or 5 substituents R^{Ar}" is also expressed as "aryl which carries 0, 1, 2, 3, 4 or 5 substituents R^{Ar}".

The definitions of the variables specified in the above formulae use collective terms which are generally representative of the respective substituents. The definition Cₙ-Cₘ gives the number of carbon atoms possible in each case in the respective substituent or substituent moiety.

The term "halogen" denotes in each case fluorine, bromine, chlorine or iodine.

The term "alkyl" as used herein refers to saturated straight-chain or branched hydrocarbon radicals having 1 to 30 ("C₁-C₃₀-alkyl"), 1 to 18 ("C₁-C₁₈-alkyl"), 1 to 12 ("C₁-C₁₂-alkyl"), 1 to 8 ("C₁-C₈-alkyl"), 1 to 6 ("C₁-C₆-alkyl"), 1 to 4 ("C₁-C₄-alkyl") or 1 to 2 ("C₁-C₂-alkyl") carbon atoms. C₁-C₂-Alkyl is methyl or ethyl. C₁-C₄-Alkyl is additionally n-propyl, isopropyl, n-butyl, 1-methylpropyl (sec-butyl), 2-methylpropyl (isobutyl) or 1,1-dimethylethyl (tert-butyl). C₁-C₆-Alkyl is additionally also, for example, n-pentyl, 1-methylbutyl, 2-methylbutyl, 3-methylbutyl, 2,2-dimethylpropyl, 1-ethylpropyl, 1,1-dimethylpropyl, 1,2-dimethylpropyl, n-hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, or 1-ethyl-2-methylpropyl. C₁-C₆-Alkyl is additionally also, for example, n-pentyl or n-hexyl.

The term "haloalkyl" as used herein, refers to a straight-chain or branched alkyl group having 1 to 6 ("C₁-C₆-haloalkyl") carbon atoms (as specified above), where some or all of the hydrogen atoms in these groups are replaced by halogen atoms as specified above. Examples are chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl,1-fluoropropyl, 2-fluoropropyl, 3-fluoropropyl, 1,1-difluoropropyl, 2,2-difluoropropyl, 1,2-difluoropropyl, 3,3-difluoropropyl, 3,3,3-trifluoropropyl, heptafluoropropyl, 1,1,1-trifluoroprop-2-yl, 3-chloropropyl and the like.

The term "cycloalkyl" as used herein refers to mono- or bicyclic saturated hydrocarbon radicals having usually 3 to 8 ("C₃-C₈-cycloalkyl") or 3 to 6 carbon atoms ("C₃-C₆-cycloalkyl"). Examples of monocyclic radicals having 3 to 6 carbon atoms comprise cyclopropyl, cyclobutyl, cyclopentyl and cyclohexyl. Examples of monocyclic radicals having 3 to 8 carbon atoms comprise cyclopropyl, cyclobutyl, cyclopentyl, cyclohexyl, cycloheptyl and cyclooctyl. Examples of bicyclic radicals having 7 or 8 carbon atoms comprise bicyclo[2.2.1]heptyl, bicyclo[3.1.1]heptyl, bicyclo[2.2.2]octyl, bicyclo[3.3.0]octyl and bicyclo[3.2.1]octyl. Preferably, the term cycloalkyl denotes a monocyclic saturated hydrocarbon radical havong 3 to 6 carbon atoms.

The term "alkenyl" as used herein refers to monounsaturated straight-chain or branched hydrocarbyl radicals having 2 to 30 ("C₂-C₃₀-alkenyl"), for example 2 to 20 ("C₂-C₂₀-alkenyl") or 3 to 10 ("C₂-C₁₀-alkenyl") carbon atoms and one double bond in any position, for example ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "alkynyl" as used herein refers to straight-chain or branched hydrocarbyl radicals having 2 to 30 (C₂-C₃₀-alkynyl), for example 2 to 20 ("C₂-C₂₀-alkynyl") or 3 to 10 ("C₂-C₁₀-alkynyl") carbon atoms and one triple bond at any position, for example ethynyl, 1-propynyl, 2-propynyl, 1-butynyl, 2-butynyl, 3-butynyl, 1-methyl-2-propynyl.

The term "heterocyclyl" as used herein refers to a mono- or bicyclic saturated or partially unsaturated ring system having 3, 4, 5, 6, 7 or 8 ring members (C₃-C₈-heterocyclyl), comprising besides carbon atoms as ring members, one, two, three or four heteroatoms or heteroatom-containing groups selected from O, N, S, SO and S(O)₂ as ring members.

The term "aryl" as used herein refers to mono-, di- or trinuclear (monocyclic, bicyclic or tricyclic) aromatic hydrocarbyl radicals having 6 to 14 and more preferably 6 to 10 carbon atoms, which do not comprise any ring heteroatoms. Examples of aryl are especially phenyl, naphthyl, indenyl, fluorenyl, anthracenyl, phenanthrenyl, and especially phenyl or naphthyl.

The term "heteroaryl (hetaryl)" as used herein refers to a mono-, di- or trinuclear (monocyclic, bicyclic or tricyclic) aromatic ring system having 5 to 14 ring members, some of which can be derived from the aforementioned aryl, in which at least one carbon atom in the aryl base skeleton is replaced by a heteroatom. Preferred heteroatoms are N, O and S. More preferably, the heteroaryl radicals have 5 to 13 ring atoms. More preferably, the heteroaryl radicals have besides carbon atoms, one, two, three or four heteroatoms selected from O, S and N as ring members.

With a view to the use of the compound of the formula (I) according to the invention, the remarks made below concerning the variables R^{Ar}, R¹, R², R³, R⁴, R⁵, R⁶, R⁷, R⁸, R⁹ and R¹⁰ are valid both on their own and, in particular, in every possible combination with each other.

In a preferred embodiment, 1, 2, 3 or 4 of the radicals R¹, R², R³, R⁴, R⁵, R⁶, R⁷, R⁸, R⁹ and R¹⁰ is/are aryl which carries 1 or 2 cyano groups and 0, 1 or 2 radicals R^{Ar}; 0, 1, 2, 3 or 4 of the radicals R¹, R², R³, R⁴, R⁵, R⁶, R⁷, R⁸, R⁹ and R¹⁰ are aryl which is unsubstituted or carries 1, 2 or 3 radicals R^{Ar}; and the remaining radicals R¹, R², R³, R⁴, R⁵, R⁶, R⁷, R⁸, R⁹ and R¹⁰ are hydrogen. R^{Ar}, if present, has one of the meanings given above and preferably is C₁-C₁₀-alkyl, C₁-C₆-haloalkyl, C₃-C₈-cycloalkyl or phenyl which is unsubstituted or carries 1, 2 or 3 radicals R^{c}. R^{c}, if present, is as defined above.

In an even more preferred embodiment, 1, 2, 3 or 4 of the radicals R¹, R², R³, R⁴, R⁵, R⁶, R⁷, R⁸, R⁹ and R¹⁰ is/are phenyl which carries 1 or 2 cyano groups; 0, 1, 2 or 3 of the radicals R¹, R², R³, R⁴, R⁵, R⁶, R⁷, R⁸, R⁹ and R¹⁰ are phenyl which is unsubstituted or carries 1, 2 or 3 radicals R^{Ar}; and the remaining radicals R¹, R², R³, R⁴, R⁵, R⁶, R⁷, R⁸, R⁹ and R¹⁰ are hydrogen. R^{Ar}, if present, has one of the meanings mentioned as being preferred. Likewise in an even more preferred embodiment, 1, 2, 3 or 4 of the radicals R¹, R², R³, R⁴, R⁵, R⁶, R⁷, R⁸, R⁹ and R¹⁰ is/are phenyl which carries 1 or 2 cyano groups, wherein at least one of the radicals R⁷, R⁸, R⁹ and R¹⁰ is phenyl which carries 1 or 2 cyano groups; 0, 1, 2 or 3 of the radicals R¹, R², R³, R⁴, R⁵, R⁶, R⁷, R⁸, R⁹ and R¹⁰ are phenyl which is unsubstituted or carries 1, 2 or 3 radicals R^{Ar}; and the remaining radicals R¹, R², R³, R⁴, R⁵, R⁶, R⁷, R⁸, R⁹ and R¹⁰ are hydrogen. R^{Ar}, if present, has one of the meanings mentioned as being preferred.

Especially, 2, 3 or 4 of the radicals R¹, R², R³, R⁴, R⁵, R⁶, R⁷, R⁸, R⁹ and R¹⁰ is/are phenyl which carries 1 or 2 cyano groups; 1 or 2 of the radicals R¹, R², R³, R⁴, R⁵, R⁶, R⁷, R⁸, R⁹ and R¹⁰ are phenyl which is unsubstituted or carries 1, 2 or 3 radicals R^{Ar}; and the remaining radicals R¹, R², R³, R⁴, R⁵, R⁶, R⁷, R⁸, R⁹ and R¹⁰ are hydrogen. R^{Ar}, if present, has one of the meanings mentioned as being preferred.

Likewise especially, 2, 3 or 4 of the radicals R¹, R², R³, R⁴, R⁵, R⁶, R⁷, R⁸, R⁹ and R¹⁰ is/are phenyl which carries 1 or 2 cyano groups, wherein at least one of the radicals R⁸, R⁹ and R¹⁰ is phenyl which carries 1 or 2 cyano groups; 1 or 2 of the radicals R¹, R², R³, R⁴, R⁵, R⁶, R⁷, R⁸, R⁹ and R¹⁰ is/are phenyl which is unsubstituted or carries 1 or 2 or 3 radicals R^{Ar}; and the remaining radicals R¹, R², R³, R⁴, R⁵, R⁶, R⁷, R⁸, R⁹ and R¹⁰ are hydrogen. Especially, R⁷ is hydrogen. R^{Ar}, if present, has one of the meanings mentioned as being preferred.

Especially preferred are compounds of formula (I), which correspond to a compound of formula (I-A) 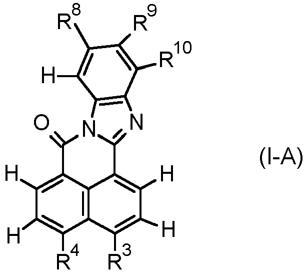 wherein
R³ and R⁴ are each independently hydrogen; phenyl; phenyl which carries 1 or 2 cyano groups; or phenyl which carries 1, 2 or 3 substituents selected from C₁-C₁₀-alkyl; and
at least one of the radicals R⁸, R⁹ and R¹⁰ is phenyl which carries 1 or 2 cyano groups; and the remaining radicals R⁸, R⁹ and R¹⁰ are selected from the group consisting of hydrogen and phenyl which is unsubstituted or carries 1, 2 or 3 substituents selected from C₁-C₁₀-alkyl.

Among the compounds of formula (I-A), preference is given to compounds, in which R⁸ and R¹⁰ have the same meaning and are both phenyl which carries 1 or 2 cyano groups. In particular, R⁸ and R¹⁰ have the same meaning and R⁹ is hydrogen.

A particular preferred embodiment of the invention relates to compounds of the formula (I-A), wherein
- R³ and R⁴: are each independently selected from hydrogen; phenyl; phenyl which carries 1 or 2 cyano groups; and phenyl which carries 1, 2 or 3 C₁-C₁₀-alkyl substituents; in particular hydrogen, phenyl or phenyl which carries 1 cyano group;
- R⁸: is phenyl which carries 1 or 2 cyano groups; phenyl; or phenyl which carries 1, 2 or 3 substituents C₁-C₁₀-alkyl;
- R⁹: is hydrogen; and
- R¹⁰: is phenyl which carries 1 or 2 cyano groups; phenyl; or phenyl which carries 1, 2 or 3 substituents C₁-C₁₀-alkyl.

A more particular preferred embodiment of the invention relates to compounds of the formula (I-A), wherein
- R³: is phenyl; phenyl which carries 1 cyano group; or phenyl which carries 1 substituent selected from C₁-C₁₀-alkyl; in particular phenyl which carries 1 cyano group;
- R⁴: is hydrogen;
- R⁸ and R¹⁰: are each phenyl which carries 1 cyano group;
- R⁹: is hydrogen.

A further especially preferred embodiment of the invention relates to compounds of the formula (I-A), wherein
- R³: hydrogen;
- R⁴: is phenyl; phenyl which carries 1 cyano group; or phenyl which carries 1 substituent selected from C₁-C₁₀-alkyl; in particular phenyl which carries 1 cyano group;
- R⁸ and R¹⁰: are each phenyl which carries 1 cyano group;
- R⁹: is hydrogen.

Examples of preferred compounds of formula (I-A) are the compounds of formulae (I-A.1), (I-A.2) (I-A.3) and (I-A.4)

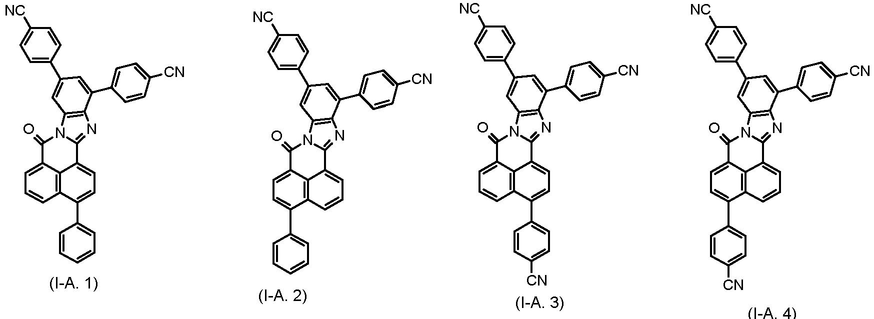

Compounds of the formula I and mixtures thereof can be prepared in analogy to standard methods, for example as described in WO 2012/168395, especially on pages 64 - 81, WO 2015/019270, on pages 21 - 30 or as described in the experimental section of this application.

As an example, the preparation of compounds of formula (I), where at least one of the radicals R⁷, R⁸, R⁹ and R¹⁰ ,independently of each other, is aryl which carries one, two or three cyano groups and 0, 1, 2, 3 or 4 substituents R^{Ar} and the remaining radicals R⁷, R⁸, R⁹ and R¹⁰ independently of each other are selected from hydrogen and aryl which is unsubstituted or carries 1, 2, 3, 4 or 5 substituents R^{Ar}; and R¹, R², R³, R⁴, R⁵ and R⁶ ,independently of each other, are selected from hydrogen and aryl which carries 0, 1, 2 or 3 cyano groups and 0, 1, 2, 3, 4 or 5 substituents R^{Ar} is described below in detail. The preparation comprises the steps:
(i) a diamine of formula (V) 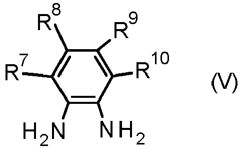 wherein
   at least one of the radicals R⁷, R⁸, R⁹ or R¹⁰ is aryl which carries one, two or three cyano groups and 0, 1, 2, 3 or 4 substituents R^{Ar}, and the remaining radicals R⁷, R⁸, R⁹ or R¹⁰ are hydrogen or aryl which is unsubstituted or carries 1, 2, 3, 4 or 5 substituents R^{Ar};
   is reacted with a 1,8-naphthalic anhydride of formula (VI) 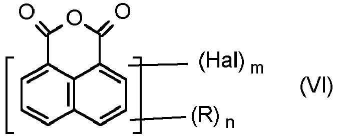 wherein
   - Hal: is bromine or chlorine;
   - R: independently of each other is selected from hydrogen and aryl which carries 0, 1, 2 or 3 cyano groups and 0, 1, 2, 3, 4 or 5 substituents R^{Ar};
   - m: is 1, 2, 3, 4, 5 or 6;
   - n: is 0, 1, 2, 3, 4 or 5;
   with the proviso that the sum of m + n is 6
   to obtain a compound of formula (VII) 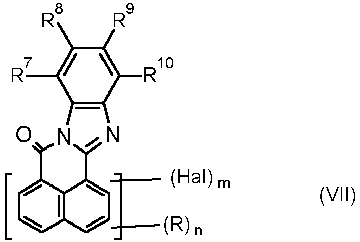 wherein (R)ₙ, (Hal)ₘ, R⁷, R⁸, R⁹ and R¹⁰ are as defined above;
(ii) the compound of formula (VII) obtained in step (i) is subjected to cross-coupling with an organometallic compound of formula (VIII)

   Ar-Met (VIII)

   in which
   - Ar: is aryl which carries 0, 1, 2 or 3 cyano groups and 0, 1, 2, 3, 4 or 5 substituents R^{Ar}; and
   - Met: is B(OH)₂, B(OR')(OR"), Zn-Hal' or Sn(R*)₃,
   in which
   - R' and R": are each independently hydrogen, C₁-C₃₀-alkyl, C₅-C₃-cycloalkyl, C₆-C₁₄-aryl or heteroaryl, or R' and R" together are C₂-C₄-alkylene which optionally bears 1, 2, 3, 4, 5, 6, 7 or 8 substituents selected from C₁-C₄-alkyl, C₅-C₃-cycloalkyl, C₆-C₁₄-aryl and heteroaryl,
   - Hal': is chlorine or bromine and
   - R*: is C₁-C₈-alkyl or phenyl,
   in the presence of a transition metal catalyst to obtain the compound of formula I 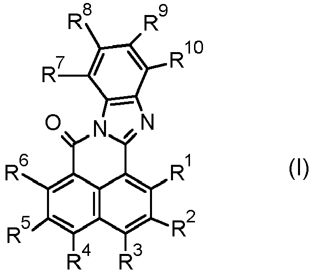 where
   at least one of the radicals R⁷, R⁸, R⁹ and R¹⁰ independently of each other is aryl which carries one, two or three cyano groups and 0, 1, 2, 3 or 4 substituents R^{Ar} and the remaining radicals R⁷, R⁸, R⁹ and R¹⁰ independently of each other are selected from hydrogen and aryl which is unsubstituted or carries 1, 2, 3, 4 or 5 substituents R^{Ar}; and
   R¹, R², R³, R⁴, R⁵ and R⁶ independently of each other are selected from hydrogen and aryl which carries 0, 1, 2 or 3 cyano groups and 0, 1, 2, 3, 4 or 5 substituents R^{Ar}.

### Step (i)

The reaction is preferably carried out in the presence of a polar aprotic solvent. Suitable polar aprotic solvents are nitrogen heterocycles such as pyridine, pyrimidine, quinoline, isoquinoline, quinaldine, N-methylpiperidine, N-methylpiperidone and N-methylpyrrolidone (NMP) or alkyl aryl ethers such as anisole.

The reaction is advantageously effected in the presence of an imidization catalyst. Suitable imidization catalysts are organic and inorganic acids, for example formic acid, acetic acid, propionic acid and phosphoric acid. Suitable imidization catalysts are also organic and inorganic salts of transition metals, such as zinc, iron, copper and magnesium. Examples of these include zinc acetate, zinc propionate, zinc oxide, iron(II) acetate, iron(III) chloride, iron(II) sulfate, copper(II) acetate, copper(II) oxide and magnesium acetate. The molar ratio of anhydride to imidization catalyst is generally 1.2:1 to 1:1.2.

The reaction temperature is generally ambient temperature to 200°C, preferably 120°C to 160°C.

Compounds of formulae (V) and (VI) are commercially available or can be prepared according to standard methods. Compounds of formula (V) can be prepared according to literature methods, e.g. starting from bromination of 2-nitroaniline. The obtained brominated 2-nitroaniline is treated with a cyanoarylboronic acid in the sense of a Suzuki coupling. Reduction of the aromatic nitro group to an amine group using zinc chloride affords the compound of formula (V).

The compound of formula (VII) obtained in step (i) is generally used for the subsequent reaction without further purification and/or separation.

A skilled person will readily appreciate that the reaction in step (i) may afford regioisomers depending on the substitution pattern on the naphthalene skeleton of the 1,8-naphthalic anhydride of formula (VI) and the substitution pattern of the diamine of formula (V).

### Step (ii)

The compound of formula (VII) obtained in step (i) is subjected to a cross-coupling with an organometallic compound of formula (VIII).

Preference is given to effecting the reaction in the presence of catalytically active amounts of a transition metal of transition group VIII of the Periodic Table (group 10 according to IUPAC), for example nickel, palladium or platinum, especially in the presence of a palladium catalyst. Suitable catalysts are, for example, palladium-phosphine complexes such as tetrakis(triphenylphosphine)palladium(0), PdCl₂(o-tolyl₃P)₂, bis(triphenylphosphine)palladium(II) chloride, the [1,1'-bis(diphenyl-phosphino)ferrocene]palladium(II) chloride-dichloromethane complex, bis[1,2-bis(diphenylphosphino)ethane]palladium(0) and [1,4-bis(diphenylphosphino)-butane]palladium(II) chloride, palladium on activated carbon in the presence of phosphine compounds, and palladium(II) compounds such as palladium(II) chloride or bis(acetonitrile)palladium(II) chloride in the presence of phosphine compounds, such as triphenylphosphine, 1,1'-bis(diphenylphosphino)ferrocene, 1,2-bis(diphenylphosphino)-ethane, 1,3-bis(diphenylphosphino)propane and 1,4-bis(diphenylphosphino)butane. The amount of catalyst is typically 10 to 150 mol%, based on the compounds of the formula (VII).

Especially suitable organometallic compounds (VIII) are an appropriately substituted arylboronic acid and arylboronic esters (compounds VIII where Met = B(OH)₂ or B(OR')(OR") where R', R" = C₁-C₄-alkyl, or R' and R" together are C₂-C₄-alkylene optionally bearing 1, 2, 3 or 4 substituents selected from C₁-C₄-alkyl).

The reaction is effected under the conditions of a Suzuki coupling, as known, for example, from Suzuki et al., Chem. Rev., 1995, 95, 2457-2483 and the literature cited therein. The arylboronic acids and esters thereof are known from the literature, commercially available, or can be prepared from the corresponding arylmagnesium compounds by reaction with appropriate boric esters.

Suitable organometallic compounds (VIII) are especially also arylstannanes (compounds VIII where Met = Sn(R*)₃, where R* is especially C₁-C₄-alkyl). In that case, the reaction is effected under the conditions of a Stille coupling, as known, for example, from D. Milstein, J. K. Stille, J. Am. Chem. Soc. 1978, 100, P. 3636-3638 or V. Farina, V. Krishnamurthy, W. J. Scott, Org. React. 1997, 50, 1-652. Arylstannanes VIII can be prepared in analogy to known processes by reaction of aryllithium compounds with (R*)₃SnCl.

Suitable organometallic compounds VIII are additionally organozinc compounds (compounds VIII where Met = Zn-Hal' where Hal' = Cl, Br, especially Br). In that case, the reaction is effected under the conditions of a Negishi coupling, as known, for example, from A. Lützen, M. Hapke, Eur. J. Org. Chem., 2002, 2292-2297. Arylzinc compounds can be prepared in a manner known per se from the aryllithium compounds or from the arylmagnesium compounds by reaction with zinc salts such as zinc chloride.

The reaction of compound of formula (VII) with the organometallic compound (VIII), especially in the case of the Suzuki coupling, is effected under basic conditions. Suitable bases are alkali metal carbonates and alkali metal hydrogencarbonates such as sodium carbonate, potassium carbonate, cesium carbonate, sodium hydrogencarbonate, alkaline earth metal carbonates and alkaline earth metal hydrogencarbonates such as magnesium carbonate or magnesium hydrogencarbonate, or tertiary amines such as triethylamine, trimethylamine, triisopropylamine or N-ethyl-N-diisopropylamine.

Typically, the coupling is carried out in a solvent. Suitable solvents are organic solvents such as aromatics, e.g. toluene, ethers, e.g. dialkylether such as 1,2-dimethoxyethane, cyclic ethers such as tetrahydrofuran or 1,4-dioxane, alkyl aryl ether such as anisole, polyalkylene glycols such as diethylene glycol, carbonitriles such as acetonitrile, propionitrile, carboxamides such as dimethylformamide or dimethylacetamide. In the Suzuki coupling, the aforementioned solvents can also be used in a mixture with water; for example, the ratio of organic solvent to water may be in the range from 5:1 to 1:5.

At least one mole of the organometallic compound VIII is used per mole of halogen atom to be exchanged. It may be advantageous to use a 5 to 30% molar excess of organometallic compound of the formula V per mole of halogen atom to be exchanged.

The obtained compound of formula (I) can be purified by customary processes known to those skilled in the art, such as extraction, distillation, recrystallization, separation on suitable stationary phases, and a combination of these measures.

A skilled person will readily appreciate that the reaction affords a mixture of regioisomers of formula (I), if the starting material VII is a mixture of regioisomers. The obtained regioisomers of formula (I) may be separated and/or purified by customary processes known to those skilled in the art, such as extraction, distillation, recrystallization, separation on suitable stationary phases, and a combination of these measures.

The compound of the formula (I) or mixtures thereof as defined above can be used for a whole series of end uses for example as fluorescent colorant in color converters. Thus, it is possible to convert light produced by a white LED having a CCT between 3 000 K to 20 000 K, e.g. 6 000 K to 20 000K, or 4 500 to 20 000 K, to light having a lower CCT (i.e. warmer white light) by passing light generated by said white LED through a color converter comprising the compound of formula (I). Likewise, it is possible to convert light produced by a blue LED with a center wavelength of emission between 400 nm and 480 nm, e.g. 420 to 480 nm, to white light by passing light generated by said LED through a color converter comprising the compound of formula (I).

The invention also provides a color converter comprising in a polymeric matrix at least one naphthoylenebenzimidazolecompound of the formula (I) as defined above or a mixture thereof as fluorescent colorant. In a less prefered embodiment, inventive color converter comprise a compound of formula (I) or mixtures thereof as single phosphor component.

The spectral composition of the generated light can be better adapted for the intended use if the compound of formula (I) or mixtures thereof are used in combination with further fluorescent colorants being different from compounds of formula (I). According to a specific embodiment, the compound of formula (I) or a mixture of compounds of formula (I) are used in combination with at least one further phosphor, especially an organic fluorescent colorant. Suitable further organic fluorescent colorants are in principle all organic dyes or pigments which can absorb light and convert it to light of other wavelengths. The further colorants should be capable of being dissolved or distributed homogeneously in the polymeric matrix and should have a sufficient stability to thermal and radiative stress.

Thus, in a further aspect, the invention provides a color converter comprising in a polymeric matrix at least one naphthoylenebenzimidazolecompound of the formula (I) as defined above as fluorescent colorant and at least one further organic fluorescent colorant which is selected from
(a) an organic orange fluorescent colorant having a maximum of emission in the wavelength range of 540 to less than 600 nm, preferably 540 to 590 nm and mixtures thereof; and
(b) an organic red fluorescent colorant having a maximum of emission in the wavelength range of 600 to 670 nm and mixtures thereof;
and optionally at least one inorganic white pigment as a scattering body.

In this context, the fluorescence spectra of the organic orange fluorescent colorant(s) and organic red fluorescent colorant(s) are recorded in the polymer or polymer mixture used in the color converter in question.

The further fluorescent colorants may absorb at least part of the light of a blue LED having a center wavelength of emission between 400 to 480 nm, especially 420 nm and 480 nm.

Suitable organic orange fluorescent colorants having a maximum of emission in the wavelength range of 540 to less than 600 nm, especially 540 to 590 nm include compounds of the core-cyanated benzimidazole type, bay-unsubstituted perylene bisimide compounds and aryloxy-substituted perylene bisimides with rigid 2,2'-biphenoxy bridges.

Preferably, the orange fluorescent colorant having a maximum of emission in the wavelength range of 540 to 590 nm is selected from
(a.1) a core-cyanated naphthoylenebenzimidazolecompound of the formula (II) 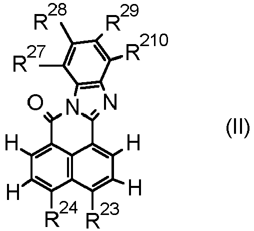 wherein
   one of R²³ or R²⁴ independently of each other is cyano and the other radical R²³ or R²⁴ is selected from cyano, phenyl, 4-cyanophenyl and phenyl which carries 1, 2 or 3 substituents selected from C₁-C₁₀-alkyl;
   R²⁷, R²⁸, R²⁹ and R²¹⁰ independently of each other are hydrogen, phenyl, 4-cyanophenyl or phenyl which carries 1, 2 or 3 substituents selected from C₁-C₁₀-alkyl,
   and mixtures thereof;
(a.2) a perylene bisimide compound of the formula (III) 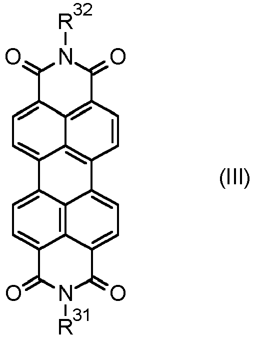 wherein
   R³¹ and R³² independently of each other are C₁-C₃₀-alkyl, C₃-C₈-cycloalkyl, aryl, heteroaryl or aryl-C₁-C₁₀-alkylene, where the (hetero)aromatic ring in the three latter radicals is unsubstituted or mono- or polysubstituted by C₁-C₁₀-alkyl;
(a.3) a perylene bisimide compound with rigid 2,2'-biphenoxy bridges of the formula (IV) 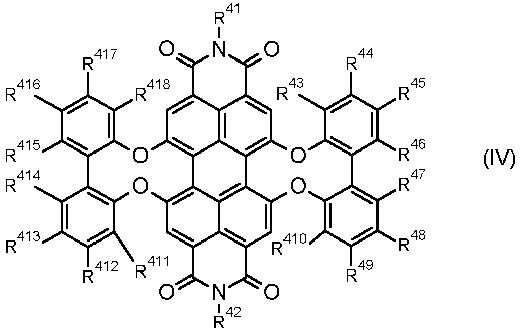 wherein
   R⁴¹ and R⁴², independently of each other, are selected from hydrogen, C₁-C₁₀-alkyl, which is unsubstituted or substituted by C₆-C₁₀-aryl which in turn is unsubstituted or substituted by 1, 2 or 3 C₁-C₁₀-alkyl, C₃-C₈-cycloalkyl, which is unsubstituted or substituted by 1, 2 or 3 C₁-C₁₀-alkyl, and C₆-C₁₀-aryl which is unsubstituted or substituted by 1, 2 or 3 C₁-C₁₀-alkyl;
   R⁴³, R⁴⁴, R⁴⁵, R⁴⁶, R⁴⁷, R⁴⁸, R⁴⁹, R⁴¹⁰, R⁴¹¹, R⁴¹², R⁴¹³, R⁴¹⁴, R⁴¹⁵, R⁴¹⁶, R⁴¹⁷ and R⁴¹⁸ independently of each other, are selected from hydrogen, halogen, C₁-C₁₈-alkyl, C₆-C₁₀-aryl, C₆-C₁₀-aryl-C₁-C₁₀-alkylene, C₆-C₁₀-aryloxy and C₆-C₁₀-arylthio, where the aryl moiety of C₆-C₁₀-aryl, C₆-C₁₀-aryl-C₁-C₁₀-alkylene, C₆-C₁₀-aryloxy and C₆-C₁₀-arylthio is unsubstituted or substituted by one or more C₁-C₁₀-alkyl
   and mixtures thereof.

Compounds of formula (II) are known from WO 2015/019270. Especially suitable compounds of formula II are those, where R²⁸ and R²¹⁰ are each phenyl and R²⁷ and R²⁹ are hydrogen. Particularly preferred is the compound of formula (II.1) corresponding to compound (27) of WO 2015/019270

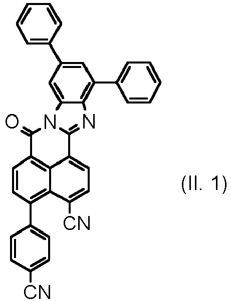

Perylen-3,4,9,10-tetracarboxylic acid diimide compounds of formula (III) are, for example, described in DE 1 130 099, US 4,379,934, US 4,446,324 or EP 0657436. Particularly preferred perylene colorants of formula (III) are those, wherein R³¹ and R³² are independently selected from C₁-C₁₀-alkyl, 2,6-di(C₁-C₁₀-alkyl)aryl and 2,4-di(C₁-C₁₀-alkyl)aryl. More preferably, R³¹ and R³² are identical. Particularly preferred is the compound of formula III.1

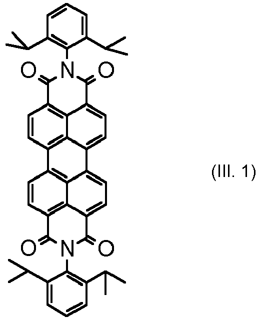

Perylene bisimide compounds which rigid 2,2'-biphenoxy bridges of formula (IV) are are subject matter of unpublished PCT/EP2017/050621 (post-published WO 2017/121833). Particularly preferred perylenbisimide compounds are those, where R⁴¹ and R⁴² are, independently of each other, selected from phenyl which is unsubstituted or substituted by 1, 2 or 3 C₁-C₆-alkyl; and R⁴³, R⁴⁴, R⁴⁵, R⁴⁶, R⁴⁷, R⁴⁸, R⁴⁹, R⁴¹⁰, R⁴¹¹, R⁴¹², R⁴¹³, R⁴¹⁴, R⁴¹⁵, R⁴¹⁶, R⁴¹⁷ and R⁴¹⁸ are each hydrogen. Particularly preferred is the compound of formula (IV.1)

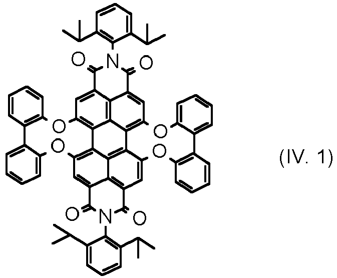

The compound of formula (IV) can be prepared by reacting the appropriate chlorinated or brominated perylene bisimide of formula (IX) 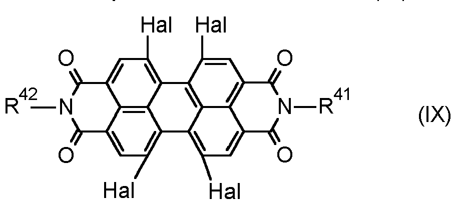 where
- Hal: is in each case bromine or in each case chlorine; and
- R⁴¹ and R⁴²: are as defined above;
with a 2,2'-biphenol compound of formula (X) and, if appropriate, an 2,2'-biphenol compound of formula (XI) 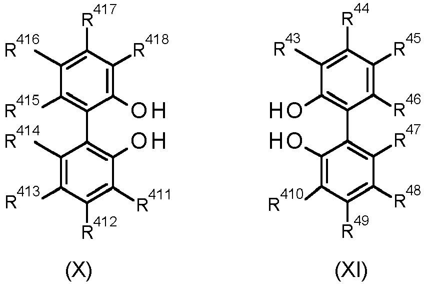 where
R⁴³, R⁴⁴, R⁴⁵, R⁴⁶, R⁴⁷, R⁴⁸, R⁴⁹, R⁴¹⁰, R⁴¹¹, R⁴¹², R⁴¹³, R⁴¹⁴, R⁴¹⁵, R⁴¹⁶, R⁴¹⁷ and R⁴¹⁸ are as defined above.

The 2,2'-biphenol of formula (XI) may also be as defined for the 2,2'-biphenol of formula (X) (if only one 2,2'-biphenol of the formula (X) is used for halogen replacement reaction).

The reaction is usually carried out in the presence of a base. Suitable bases are in particular inorganic alkali metal or alkaline earth metal bases, the alkali metal bases being particularly suitable. Examples of inorganic bases are the carbonates and hydrogencarbonates, hydroxides, hydrides and amides of alkali metals and alkaline earth metals. Preferred bases are the carbonates and hydrogencarbonates, particular preference being given to the carbonates. Preferred alkali metals are lithium, sodium, potassium and cesium; particularly suitable alkaline earth metals are magnesium and calcium. It will be appreciated that it is also possible to use base mixtures. Very particularly preferred bases are lithium carbonate, sodium carbonate, potassium carbonate and cesium carbonate.

The reaction is usually carried out in the presence of a polar, aprotic solvent. Suitable solvents are especially aliphatic carboxamides, preferably N,N-di-C₁-C₄-alkyl-C₁-C₄-carboxamides, lactams such as dimethylformamide, diethylformamide, dimethylacetamide, dimethylbutyramide and N-methyl-2-pyrrolidone (NMP), nitriles such as acetonitrile. It is also possible to use mixtures of polar, aprotic solvents. Particular preference is given to NMP.

The reaction temperature is generally within the range from room temperature to the boiling point of the solvent, preferably 40 to 160°C.

Compounds of formula (IX) can be prepared in analogy to the methods described in US 4,845,223. Compounds of formula (X) and (XI) are commercially available or can be prepared according to literature methods.

Suitable organic red fluorescent colorants having a maximum of emission in the wavelength range of 600 to 670 nm include 1,6,7,12-tetraaryloxy-substituted perylene bisimides compounds.

Preferably, the fluorescent colorant having a maximum of emission in the wavelength range of 600 to 670 nm is capable of absorbing a part of the blue color light generated from the light emitting component. In particular, the fluorescent colorant having a maximum of emission in the wavelength range of 600 to 670 nm is a perylene bisimide compound of formula (V) 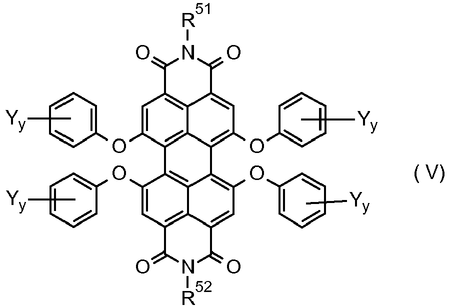 wherein
- R⁵¹ and R⁵²: independently of each other are C₁-C₁₀-alkyl, which is unsubstituted or substituted by C₆-C₁₀-aryl which in turn is unsubstituted or substituted by 1, 2 or 3 C₁-C₁₀-alkyl, C₃-C₈-cycloalkyl, which is unsubstituted or substituted by 1, 2 or 3 C₁-C₁₀-alkyl, or C₆-C₁₀-aryl which is unsubstituted or substituted by 1, 2 or 3 C₁-C₁₀-alkyl;
- Y: independently of each other and independently of each occurrence is C₁-C₁₀-alkyl, phenyl, or phenyl which is substituted by 1, 2 or 3 C₁-C₆-alkyl, C₁-C₆-alkoxy-C₁-C₆-alkyl and C₁-C₆-alkoxy; and
- y: independently of each other and independently of each occurrence is 0, 1, 2, or 3.

Examples of preferred perylene bisimide compounds of formula (V) are those, wherein R⁵¹ and R⁵² are each independently selected from C₁-C₁₀-alkyl, 2,6-di(C₁-C₁₀-alkyl)aryl and 2,4-di(C₁-C₁₀-alkyl)aryl. In particular, R⁵¹ and R⁵² are identical. Very particularly, R⁵¹ and R⁵² are each 2,6-diisopropylphenyl or 2,4-di-*tert*-butylphenyl.

In a specific embodiment, y is 0, i.e. (Y)_{y} is absent. These compounds are known from US 4,845,223. Particularly preferred is N,N'-bis(2,6-diisopropylphenyl)-1,6,7,12-tetraphenoxyperylene-3,4:9,10-tetracarboximide.

In a further specific embodiment, each y is 1 and each Y is phenyl, in particular in the ortho-position. These compounds are subject matter of unpublished EP 16192617.5.

In a still further specific embodiment, each y is 1. Y is each C₁-C₁₀-alkyl, in particular secondary C₃-C₁₀-alkyl or tert C₄-C₁₀-alkyl. Especially preferably, Y is secondary C₃-C₁₀-alkyl in the ortho/para position, for example isopropyl in the ortho-position.

Especially suitable examples are the perylene bisimide compounds of formulae (V.1), (V.2), and (V.3) shown below.

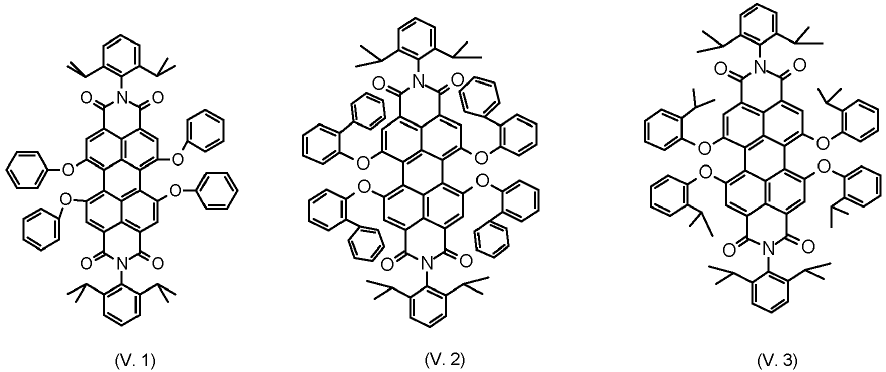

Surprisingly, it has been found that a color converter comprising at least one compound of formula (I) as defined above, at least one colorant having a maximum of emission in the wavelength range of 540 to less than 600 nm, especially 540 to 590 nm and at least one fluorescent colorant having a maximum of emission in the wavelength range of 600 to 670 nm allows to obtain white light having CRI Ra values greater than 90, preferably at least 92 and in particular greater than 95. In particular, the obtained white light has a R9 value of greater than 66, especially greater than 70 and more especially greater than 75.

Hence, in a further aspect, the invention also provides a color converter comprising
- at least one naphthoylenebenzimidazolecompound of the formula (I) as fluorescent colorant as defined above;
- at least one orange fluorescent colorant having a maximum of emission in the wavelength range of 540 to less than 600 nm, preferably 540 to 590 nm;
- at least one red fluorescent colorant having a maximum of emission in the wavelength range of 600 to 670 nm ; and
- optionally at least one inorganic white pigment as scattering body..

The number of fluorescent colorants of formula (I) to be used in combination with the orange fluorescent colorant having a maximum of emission in the wavelength range of 540 to less than 600 nm, preferably 540 to 590 nm and the red fluorescent colorant having a maximum of emission in the wavelength range of 600 to 670 nm can be any number, preferably from 1 to 5.

The number of fluorescent colorants having a maximum of emission in the wavelength range of 540 to less than 600 nm, preferably 540 to 590 nm to be used in combination with the fluorescent colorant of formula (I) and the fluorescent colorant having a center wavelength of emission in the range of 600 to 670 nm can be any number, preferably from 1 to 5.

The number of fluorescent colorants having a maximum of emission in the wavelength range of 600 to 670 nm to be used in combination with the fluorescent colorant of formula (I) and the fluorescent colorant having a maximum of emission in the wavelength range of 540 to less than 600 nm, preferably 540 to 590 nm, can be any number, preferably from 1 to 5.

Preferably, the inventive color converter comprises at least one compound of formula (I), at least one compound of formula (II) and at least one compound of formula (V). Likewise preferably, the inventive color converter comprises at least one compound of formula (I), at least one compound of formula (III) and at least one compound of formula (V). Likewise preferably, the inventive color converter comprises at least one compound of formula (I), at least one compound of formula (IV) and at least one compound of formula (V).

More preferably, the inventive color converter comprises at least one compound of formula (I-A), at least one compound of formula (II) and at least one compound of formula (V). In a specific embodiment, the color converter comprises at least one compound of formula (I-A), the compound of formula (11.1) and the compound of formula (V.1). In a further specific embodiment, the color converter comprises at least one compound of formula (I-A), the compound of formula (11.1) and the compound of formula (V.2). In a further specific embodiment, the color converter comprises at least one compound of formula (I-A), the compound of formula (11.1) and the compound of formula (V.3).

Likewise more preferably, the inventive color converter comprises at least one compound of formula (I-A), at least one compound of formula (III) and at least one compound of formula (V). In a specific embodiment, the color converter comprises at least one compound of formula (I-A), the compound of formula (III.1) and the compound of formula (V.1). In a further specific embodiment, the color converter comprises at least one compound of formula (I-A), the compound of formula (III.1) and the compound of formula (V.2). In a further specific embodiment, the color converter comprises at least one compound of formula (I-A), the compound of formula (III.1) and the compound of formula (V.3).

Likewise more preferably, the inventive color converter comprises at least one compound of formula (I-A), at least one compound of formula (IV) and at least one compound of formula (V). In a specific embodiment, the color converter comprises at least one compound of formula (I-A), the compound of formula (IV.1) and the compound of formula (V.1). In a further specific embodiment, the color converter comprises at least one compound of formula (I-A), the compound of formula (IV.1) and the compound of formula (V.2). In a further specific embodiment, the color converter comprises at least one compound of formula (I-A), the compound of formula (IV.1) and the compound of formula (V.3).

In a specific embodiment, the color converter comprises the compound of formula (I-A.1), the compound of formula (11.1) and the compound of formula (V.1).

In a further specific embodiment, the color converter comprises the compound of formula (I-A.2), the compound of formula (II.1) and the compound of formula (V.1).

In a further specific embodiment, the color converter comprises a mixture of compounds of formulae (I-A.1) and (I-A.2), the compound of formula (II.1) and the compound of formula (V.1).

In a further specific embodiment, the color converter comprises the compound of formula (I-A.3), the compound of formula (II.1) and the compound of formula (V.1).

In a further specific embodiment, the color converter comprises a compound of formula (I-A.4), the compound of formula (11.1) and the compound of formula (V.1).

In a further specific embodiment, the color converter comprises a mixture of compounds of formulae (I-A.3) and (I-A.4), the compound of formula (II.1) and the compound of formula (V.1).

In a further specific embodiment, the color converter comprises the compound of formula (I-A.1), the compound of formula (III.1) and the compound of formula (V.1).

In a further specific embodiment, the color converter comprises the compound of formula (I-A.2), the compound of formula (III.1) and the compound of formula (V.1).

In a further specific embodiment, the color converter comprises a mixture of compounds of formulae (I-A.1) and (I-A.2), the compound of formula (III.1) and the compound of formula (V.1).

In a further specific embodiment, the color converter comprises the compound of formula (I-A.3), the compound of formula (III.1) and the compound of formula (V.1).

In a further specific embodiment, the color converter comprises the compound of formula (I-A.4), the compound of formula (III.1) and the compound of formula (V.1).

In a further specific embodiment, the color converter comprises a mixture of compounds of formulae (I-A.3) and (I-A.4), the compound of formula (III.1) and the compound of formula (V.1).

In a further specific embodiment, the color converter comprises the compound of formula (I-A.1), the compound of formula (IV.1) and the compound of formula (V.1).

In a further specific embodiment, the color converter comprises the compound of formula (I-A.2), the compound of formula (IV.1) and the compound of formula (V.1).

In a further specific embodiment, the color converter comprises a mixture of compounds of formulae (I-A.1) and (I-A.2), the compound of formula (IV.1) and the compound of formula (V.1).

In a further specific embodiment, the color converter comprises the compound of formula (I-A.3), the compound of formula (IV.1) and the compound of formula (V.1).

In a further specific embodiment, the color converter comprises the compound of formula (I-A.4), a compound of formula (IV.1) and a compound of formula (V.1).

In a further specific embodiment, the color converter comprises a mixture of compounds of formulae (I-A.3) and (I-A.4), the compound of formula (IV.1) and the compound of formula (V.1).

In a further specific embodiment, the color converter comprises the compound of formula (I-A.1), the compound of formula (II.1) and the compound of formula (V.2).

In a further specific embodiment, the color converter comprises the compound of formula (I-A.2), the compound of formula (II.1) and the compound of formula (V.2).

In a further specific embodiment, the color converter comprises a mixture of compounds of formulae (I-A.1) and (I-A.2), the compound of formula (II.1) and the compound of formula (V.2).

In a further specific embodiment, the color converter comprises the compound of formula (I-A.3), the compound of formula (II.1) and the compound of formula (V.2).

In a further specific embodiment, the color converter comprises the compound of formula (I-A.4), the compound of formula (II.1) and the compound of formula (V.2).

In a further specific embodiment, the color converter comprises a mixture of compounds of formulae (I-A.3) and (I-A.4), the compound of formula (II.1) and the compound of formula (V.2).

In a further specific embodiment, the color converter comprises the compound of formula (I-A.1), the compound of formula (III.1) and the compound of formula (V.2).

In a further specific embodiment, the color converter comprises the compound of formula (I-A.2), the compound of formula (111.1) and the compound of formula (V.2).

In a further specific embodiment, the color converter comprises a mixture of compounds of formulae (I-A.1) and (I-A.2), the compound of formula (III.1) and the compound of formula (V.2).

In a further specific embodiment, the color converter comprises the compound of formula (I-A.3), the compound of formula (III.1) and the compound of formula (V.2).

In a further specific embodiment, the color converter comprises the compound of formula (I-A.4), the compound of formula (111.1) and the compound of formula (V.2).

In a further specific embodiment, the color converter comprises a mixture of compounds of formulae (I-A.3) and (I-A.4), the compound of formula (III.1) and the compound of formula (V.2).

In a further specific embodiment, the color converter comprises the compound of formula (I-A.1), the compound of formula (IV.1) and the compound of formula (V.2).

In a further specific embodiment, the color converter comprises the compound of formula (I-A.2), the compound of formula (IV.1) and the compound of formula (V.2).

In a further specific embodiment, the color converter comprises a mixture of compounds of formulae (I-A.1) and (I-A.2), the compound of formula (IV.1) and the compound of formula (V.2).

In a further specific embodiment, the color converter comprises the compound of formula (I-A.3), the compound of formula (IV.1) and the compound of formula (V.2).

In a further specific embodiment, the color converter comprises the compound of formula (I-A.4), the compound of formula (IV.1) and the compound of formula (V.2).

In a further specific embodiment, the color converter comprises a mixture of compounds of formulae (I-A.3) and (I-A.4), the compound of formula (IV.1) and the compound of formula (V.2).

In a further specific embodiment, the color converter comprises the compound of formula (I-A.1), the compound of formula (II.1) and the compound of formula (V.3).

In a further specific embodiment, the color converter comprises the compound of formula (I-A.2), the compound of formula (II.1) and the compound of formula (V.3).

In a further specific embodiment, the color converter comprises a mixture of compounds of formulae (I-A.1) and (I-A.2), the compound of formula (II.1) and the compound of formula (V.3).

In a further specific embodiment, the color converter comprises the compound of formula (I-A.3), the compound of formula (II.1) and the compound of formula (V.3).

In a further specific embodiment, the color converter comprises the compound of formula (I-A.4), a compound of formula (II.1) and a compound of formula (V.3).

In a further specific embodiment, the color converter comprises a mixture of compounds of formulae (I-A.3) and (I-A.4), the compound of formula (II.1) and the compound of formula (V.3).

In a further specific embodiment, the color converter comprises the compound of formula (I-A.1), the compound of formula (III.1) and the compound of formula (V.3).

In a further specific embodiment, the color converter comprises the compound of formula (I-A.2), the compound of formula (111.1) and the compound of formula (V.3).

In a further specific embodiment, the color converter comprises a mixture of compounds of formulae (I-A.1) and (I-A.2), the compound of formula (III.1) and the compound of formula (V.3).

In a further specific embodiment, the color converter comprises the compound of formula (I-A.3), the compound of formula (111.1) and the compound of formula (V.3).

In a further specific embodiment, the color converter comprises the compound of formula (I-A.4), the compound of formula (111.1) and the compound of formula (V.3).

In a further specific embodiment, the color converter comprises a mixture of compounds of formulae (I-A.3) and (I-A.4), the compound of formula (III.1) and the compound of formula (V.3).

In a further specific embodiment, the color converter comprises the compound of formula (I-A.1), the compound of formula (IV.1) and the compound of formula (V.3).

In a further specific embodiment, the color converter comprises the compound of formula (I-A.2), the compound of formula (IV.1) and the compound of formula (V.3).

In a further specific embodiment, the color converter comprises a mixture of compounds of formulae (I-A.1) and (I-A.2), the compound of formula (IV.1) and the compound of formula (V.3).

In a further specific embodiment, the color converter comprises the compound of formula (I-A.3), the compound of formula (IV.1) and the compound of formula (V.3).

In a further specific embodiment, the color converter comprises the compound of formula (I-A.4), the compound of formula (IV.1) and the compound of formula (V.3).

In a further specific embodiment, the color converter comprises a mixture of compounds of formulae (I-A.3) and (I-A.4), the compound of formula (IV.1) and the compound of formula (V.3).

The concentration of the organic fluorescent colorant in the polymer matrix is set as a function of the thickness of the color converter and the type of polymer. If a thin polymer layer is used, the concentration of the organic fluorescent colorant(s) is generally higher than in the case of a thick polymer layer.

Typically, the amount of organic fluorescent colorant in the polymer also depends on the correlated color temperature CCT to be achieved. A skilled person will appreciate that by increasing the concentration of organic fluorescent colorant of formula I and the concentration of the further organic fluorescent colorant(s), the light emitted from the LED is tuned to longer wavelength to obtain white light with a targeted CCT.

Typically, the concentration of the at least one inventive organic fluorescent colorant of formula I is 0.001 to 0.5% by weight, preferably 0.005 to 0.3% by weight, most preferably 0.01 to 0.2% by weight, based in each case on the amount of polymer used.

Typically, the concentration of the at least one organic orange fluorescent colorant having a maximum of emission in the wavelength range of 540 to less than 600 nm is 0.0001 to 0.2% by weight, preferably 0.001 to 0.01% by weight, based on the amount of polymer used.

Typically, the concentration of the at least one organic red fluorescent colorant having a maximum of emission in the wavelength range of 600 to 670 nm is 0.0001 to 0.2% by weight, preferably 0.001 to 0.01% by weight, based on the amount of polymer used.

The ratio of the at least one inventive organic fluorescent colorant of formula I to the at least one organic orange fluorescent colorant having a maximum of emission in the wavelength range of 540 to 590 nm is typically in the range from 9:1 to 50:1, preferably 10:1 to 40:1. Examples are 10:1 to 35: 1 or 10:1 to 25:1.

The ratio of the at least one inventive organic fluorescent colorant of formula I to the at least one organic red fluorescent colorant having a maximum of emission in the wavelength range of 600 to 670 nm is typically in the range from 2:1 to 20:1, preferably 3:1 to 15:1.

The ratio of the fluorescent colorant of formula (I) to the sum of the one organic orange fluorescent colorant having a maximum of emission in the wavelength range of 540 to les than 600 nm and the at least one organic red fluorescent colorant having a maximum of emission in the wavelength range of 600 to 670 nm is typically in the range from 2:1 to 1:10, preferably 1:1 to 1:8, more preferably 1:2 to 1:7. A skilled person will readily appreciate that the ratio of the colorants depends on the chosen light source. For a targeted CCT, the ratio of inventive colorant to orange colorant having a maximum of emission in the wavelength range of 540 to 590 nm and the ratio of inventive colorant to red colorant having a maximum of emission in the wavelength range of 600 to 670 nm, respectively, is greater, if the light is generated by a blue LED with a center wavelength of emission between 420 nm and 480 nm in comparison to the corresponding ratio of inventive colorant to yellow colorant and red colorant, respecitvely, if the light is generated by a white LED having a CCT between 6 000 to 20 000 K.

The polymeric matrix of the color converter according to the invention consists essentially of polystyrene, polycarbonate, polymethylmethacrylate, polyvinylpyrrolidone, polymethacrylate, polyvinyl acetate, polyvinyl chloride, polybutene, silicone, polyacrylate, epoxy resin, polyvinyl alcohol, poly(ethylene vinylalcohol)-coplymer (EVA, EVOH), polyacrylonitrile, polyvinylidene chloride (PVDC), polystyrene acrylonitrile (SAN), polybutylene terephthalate (PBT), polyethylene terephthalate (PET), polyvinyl butyrate (PVB), polyvinyl chloride (PVC), polyamides, polyoxymethylenes, polyimides, polyetherimides or mixtures thereof.

Especially, the at least one polymer consists essentially of polystyrene, polycarbonate, polyethylene terephthalate or mixtures thereof.

Polystyrene is understood here to mean, inter alia, all homo- or copolymers which result from polymerization of styrene and/or derivatives of styrene. Derivatives of styrene are, for example, alkylstyrenes such as alpha-methylstyrene, ortho-, meta-, para-methylstyrene, para-butylstyrene, especially para-tert-butylstyrene, alkoxystyrene such as para-methoxystyrene, para-butoxystyrene, para-tert-butoxystyrene. In general, suitable polystyrenes have a mean molar mass Mn of 10 000 to 1 000 000 g/mol (determined by GPC), preferably 20 000 to 750 000 g/mol, more preferably 30 000 to 500 000 g/mol.

In a preferred embodiment, the matrix of the color converter consists essentially or completely of a homopolymer of styrene or styrene derivatives. More particularly, the polymer consists of polystyrene.

In a further preferred embodiments of the invention, the matrix consists essentially or completely of a styrene copolymer, which are likewise regarded as polystyrene in the context of this application. Styrene copolymers may comprise, as further constituents, for example, butadiene, acrylonitrile, maleic anhydride, vinylcarbazole or esters of acrylic, methacrylic or itaconic acid as monomers. Suitable styrene copolymers generally comprise at least 20% by weight of styrene, preferably at least 40% and more preferably at least 60% by weight of styrene. In another embodiment, they comprise at least 90% by weight of styrene.

Preferred styrene copolymers are styrene-acrylonitrile copolymers (SAN) and acrylonitrile-butadiene-styrene copolymers (ABS), styrene-1,1'-diphenylethene copolymers, acrylic ester-styrene-acrylonitrile copolymers (ASA), methyl methacrylate-acrylonitrile-butadiene-styrene copolymers (MABS). A further preferred polymer is alpha-methylstyrene-acrylonitrile copolymer (AMSAN). The styrene homo- or copolymers can be prepared, for example, by free-radical polymerization, cationic polymerization, anionic polymerization or under the influence of organometallic catalysts (for example Ziegler-Natta catalysis). This can lead to isotactic, syndiotactic or atactic polystyrene or copolymers. They are preferably prepared by free-radical polymerization. The polymerization can be performed as a suspension polymerization, emulsion polymerization, solution polymerization or bulk polymerization. The preparation of suitable polystyrenes is described, for example, in Oscar Nuyken, Polystyrenes and Other Aromatic Polyvinyl Compounds, in Kricheldorf, Nuyken, Swift, New York 2005, p. 73-150 and references cited therein; and in Elias, Macromolecules, Weinheim 2007, p. 269-275.

In another preferred embodiment, the polymer consists of polyethylene terephthalate. Polyethylene terephthalate is obtainable by condensation of ethylene glycol with terephthalic acid.

Likewise more particularly, the polymer consists of polycarbonate. Polycarbonates are polyesters of carbonic acid with aromatic or aliphatic dihydroxyl compounds. Preferred dihydroxyl compounds are, for example, methylenediphenylenedihydroxyl compounds, for example bisphenol A. One means of preparing polycarbonates is the reaction of suitable dihydroxyl compounds with phosgene in an interfacial polymerization. Another means is the reaction with diesters of carbonic acid such as diphenyl carbonate in a condensation polymerization. The preparation of suitable polycarbonates is described, for example, in Elias, Macromolecules, Weinheim 2007, p. 343-347.

In a preferred embodiment, polymers which have been polymerized with exclusion of oxygen are used. Preferably, the monomers during the polymerization comprised a total of not more than 1000 ppm of oxygen, more preferably not more than 100 ppm and especially preferably not more than 10 ppm.

In one embodiment of the invention, suitable polymers are transparent polymers. In another embodiment, suitable polymers are opaque polymers.

The polymers mentioned above serve as a matrix material for the compound of formula (I) and mixtures thereof and, if present, other wavelenght converting materials. For example, other wavelength converting materials are the fluorescent colorants having a maximum of emission in the wavelength range of 540 to less than 600 nm and mixtures thereof as defined above and the fluorescent colorants having a maximum of emission in the wavelength range of 600 to 670 nm and mixtures thereof as defined above. The inventive fluorescent colorant(s) but, optionally also the other wavelength converting material may either be dissolved in the polymer or may be in the form of a homogeneously distributed mixture. In a preferred embodiment, the fluorescent colorant of formula (I) or a mixture therof, the fluorescent colorants having a maximum of emission in the wavelength range of 540 to less than 600 nm and fluorescent colorants having a maximum of emission in the wavelength range of 600 to 670 nm are dissolved in the polymer.

Suitable polymers may comprise, as further constituents, additives such as flame retardants, antioxidants, light stabilizers, UV absorbers, free-radical scavengers, antistats. Stabilizers of this kind are known to those skilled in the art.

Suitable antioxidants or free-radical scavengers are, for example, phenols, especially sterically hindered phenols such as butylhydroxyanisole (BHA) or butylhydroxytoluene (BHT), or sterically hindered amines (HALS). Stabilizers of this kind are sold, for example, by BASF under the Irganox® trade name. In some cases, antioxidants and free-radical scavengers can be supplemented by secondary stabilizers such as phosphites or phosphonites, as sold, for example, by BASF under the Irgafos® trade name.

Suitable UV absorbers are, for example, benzotriazoles such as 2-(2-hydroxyphenyl)-2H-benzotriazole (BTZ), triazines such as (2-hydroxyphenyl)-s-triazine (HPT), hydroxybenzophenones (BP) or oxalanilides. UV absorbers of this kind are sold, for example, by BASF under the Uvinul® trade name.

In a preferred embodiment of the invention, suitable polymers do not comprise any antioxidants or free-radical scavengers.

Further, the color converter may comprise an inorganic luminescent material or a plurality of inorganic luminescent materials. The inorganic phosphor material is preferably selected from garnets, silicates, sulfides, nitrides and oxynitrides. Particularly preferred among these are those selected from garnets, silicates, sulfides, nitrides and oxynitrides. Suitable examples of garnets, silicates, sulfides, nitrides and oxynitrides are compiled in table I below:

**Table I:**

| Class | Compounds | Excitation Peak nm | Emission Peak nm | Reference |
|---|---|---|---|---|
| Garnets | • YAG:Ce | 460-470 | 550 | US 5,998,925 |
| | • (Y₃Al₅O₁₂:Ce) | | | |
| | • (Y, Gd, Tb,Lu)₃Al₅O₁₂:Ce | | | |
| | • TAG:Ce (Tb₃Al₅O₁₂:Ce) | 460-470 | 575 | US 6,669,866, US 6,812,500, US 6,576,930, US6,0060,861, US 6,245,259, US 6,765,237 |
| Silicates | • Eu-doped Silicates | <460 | 510 to 610 | US 7,311,858, US 7,267,787 |
| | - A₂Si(OD)₄:Eu with A = Sr, Ba, Ca, Mg, Zn and D = F, Cl, S, N, Br | | | |
| | - (SrBaCa)₂SiO₄:Eu | | | US 6,809,347, US 6,943,380 |
| | - Sr₃SiO₅ | | | |
| | - Ba₂MgSi₂O₇:Eu²⁺; | | | US 6,429,583 |
| | - Ba₂SiO₄:Eu²⁺ | | | WO 02/11214 |
| | - (Ca,Ce)₃(Sc, Mg)₂Si₃O₁₂ | | | |
| Sulfides | • (Ca, Sr)S:Eu | <460 | 615-660 | |
| Nitrides | • (CaAlSiN₃:Eu²) | 455 | red | |
| | • (Sr,Ca)AlSiN₃:Eu²⁺ | | orange | WO 2005052087 |
| Oxy- | • SiAlON:Ce | 300-580 | 490 | |
| nitrides | • β-SiAlON:Eu | | 540 | |
| | • Ca-alpha-SiAlON:Eu (Ba₃Si₆O₁₂N₂:Eu) | | 585-595 | |
| | General formula | | | |
| | CaₓEu_{y}(Si,Al)₁₂(O, N)₁₆ | | | |

The inventive color converter may further comprise at least one quantum dot. Suitable quantum dots are nanocrystals of a semiconductor material having a diameter of about 20 nm or less. The quantum dot may include one of a Si-based nanocrystal, a group II-VI compound semiconductor nanocrystal, a group III-V compound semiconductor nanocrystal, a group IV-VI compound nanocrystal and a mixture thereof. The group II-VI compound semiconductor nanocrystal may include one selected from a group consisting of CdS, CdSe, CdTe, ZnS, ZnSe, ZnTe, HgS, HgSe, HgTe, CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HggZnTe, CdZnSeS, CdZnSeTe, CdZnSTe, CdHgSeS, CdHgSeTe, CdHgSTe, HgZnSeS, HgZnSeTe and HgZnSTe. The group III-V compound semiconductor nanocrystal may include one selected from a group consisting of GaN, GaP, GaAs, AIN, AlP, AlAs, InN, InP, InAs, GaNP, GaNAs, GaPAs, AINP, AINAs, AlPAs, InNP, InNAs, InPAs, GaAINP, GaAINAs, GaAlPAs, GalnNP, GalnNAs, GalnPAs, InAINP, InAINAs, and InAIPAs. The IV-VI compound semiconductor nano crystal may be SnTe.

To synthesize a nanocrystal in form of a quantum dot, quantum dots may be prepared by vapor deposition such as metal organic chemical vapor deposition or molecular beam epitaxy, or by a wet chemical process in which a crystal is grown by adding one or more precursors into an organic solvent.

According to one embodiment of the invention, inventive color converters have a laminate structure. They may either have a monolayer structure or a multilayer structure, generally composed of a plurality of polymer layers comprising one or more fluorescent colorants and/or scattering bodies. If the color converter has a multilayer structure, one layer may comprise the organic orange fluorescent colorant having a maximum of emission in the wavelength range of 540 to below 600 and the organic red fluorescent colorant having a maximum of emission in the wavelength range of 600 to 670 nm and another layer comprises at least one fluorescent colorant of formula (I) as defined above.

In one embodiment, the organic orange fluorescent colorant having a maximum of emission in the wavelength range of 540 to below 600 and the organic red fluorescent colorant having a maximum of emission in the wavelength range of 600 to 670 nm is present in the layer of the color converter facing the LED. In another embodiment, the at least one compouond of formula (I) is present in the layer of the color converter facing the LED.

According to any of the above embodiments, the color converter can additionally comprises at least one inorganic white pigment as a scattering body.

In a preferred embodiment, at least one of the layers or matrices comprising organic fluorescent colorants comprises scattering bodies for light.

Suitable scattering bodies are inorganic white pigments, for example titanium dioxide, barium sulphate, lithopone, zinc oxide, zinc sulphide, calcium carbonate with a mean particle size to DIN 13320 of 0.01 to 10 µm, preferably 0.1 to 1 µm, more preferably 0.15 to 0.4 µm, especially scattering bodies based on TiO₂.

Scattering bodies are included typically in an amount of 0.01 to 2.0% by weight, preferably 0.05 to 1% by weight, more preferably 0.1 to 0.5% by weight, based in each case on the polymer of the layer comprising scattering bodies.

In a preferred embodiment, the color converter has a two-layer structure wherein one layer comprises the organic fluorescent colorant having a maximum of emission in the wavelength range of 540 to below 600 and the organic fluorescent colorant having a maximum of emission in the wavelength range of 600 to 670 nm and the other layer comprises at least one fluorescent colorant of formula (I), with the layer comprising the organic fluorescent colorant having a maximum of emission in the wavelength range of 540 to below 600 and the organic fluorescent colorant having a maximum of emission in the wavelength range of 600 to 670 nm facing the blue light source. In this embodiment, both layers comprise TiO₂ as a scattering body.

In one embodiment, the color converters consist of a plurality of polymer layers which have been laminated together to form a composite and wherein the various fluorescent colorants and/or scattering bodies may be present in different polymer layers.

If inventive color converters comprise more than one organic fluorescent colorant, it is possible in one embodiment of the invention for a plurality of fluorescent colorants to be present alongside one another in one layer.

In another embodiment, the various fluorescent colorants are present in various layers.

In a further embodiment, at least one polymer layer of the color converter has been mechanically reinforced with glass fibers.

Inventive color converters may be in any desired geometric arrangement. The color converters may, for example, be in the form of films, sheets or plaques. Equally, the matrix containing organic fluorescent colorants may be in droplet form or hemispherical form or in the form of lenses with convex and/or concave, flat or spherical surfaces. "Casting" refers to the embodiment where LEDs or components comprising LEDs are cast or enveloped fully with a polymer comprising organic fluorescent colorant. In one embodiment of the invention, the polymer layers (matrices) comprising at least one organic fluorescent colorant are 25 to 400 micrometers (µm) thick, preferably 35 to 300 µm and particularly 50 to 250 µm.

In another embodiment, the polymer layers comprising organic fluorescent colorants are 0.2 to 5 millimeters thick, preferably 0.3 to 3 mm and more preferably 0.4 to 1 mm.

If the color converters consist of one layer or they have a laminate structure, the individual layers, in a preferred embodiment, are continuous and do not have any holes or interruptions.

Inventive color converters may optionally comprise further constituents, such as a backing layer.

Backing layers serve to impart mechanical stability to the color converter. The type of material for the backing layers is not crucial, provided that it is transparent and has the desired mechanical strength. Suitable materials for backing layers are, for example, glass or transparent rigid organic polymers, such as polycarbonate, polystyrene or polymethacrylates or polymethyl methacrylates.

Backing layers generally have a thickness of 0.1 mm to 10 mm, preferably 0.2 mm to 5 mm, more preferably 0.3 mm to 2 mm.

In one embodiment of the invention, inventive color converters have at least one barrier layer against oxygen and/or water, as disclosed in WO 2012/152812. Examples of suitable barrier materials for barrier layers are, for example, glass, quartz, metal oxides, SiO₂, a multilayer system composed of alternating layers of Al₂O₃ and SiO₂ layers, titanium nitride, SiO₂/metal oxide multilayer materials, polyvinyl alcohol, polyacrylonitrile, polyvinylidene chloride (PVDC), liquid crystal polymers (LCP), polystyrene-acrylonitrile (SAN), polybutylene terephthalate (PBT), polybutylene naphthalate (PBN), polyethylene terephthalate (PET), polyethylene naphthalate (PEN), polyvinyl butyrate (PBT), polyvinyl chloride (PVC), polyamides, polyoxymethylenes, polyimides, polyetherimides, epoxy resins, polymers which derive from ethylene-vinyl acetate (EVA) and polymers which derive from ethylene-vinyl alcohol (EVOH).
A preferred material for barrier layers is glass or a multilayer system composed of alternating layers of Al₂O₃ and SiO₂ layers.

Preferably, suitable barrier layers have low permeability for oxygen.

More preferably, suitable barrier layers have low permeability for oxygen and water.

The combination of organic fluorescent colorants in the polymeric matrix as described above may be excited by a source of blue light such as a blue LED. Likewise, the above described combination of organic fluorescent colorants in the polymeric matrix may be excited by a source of white light such as a white LED.

Hence, a further aspect, the present invention relates to the use of a color converter as defined above for conversion of light generated by a blue LED with a center wavelength of emission between 400 nm and 480 nm, preferably 420 to 480 nm, to provide white light. Suitable LEDs are, for example, those based on gallium nitride (GaN) or indium gallium nitride (InGaN). Likewise possible is their use for conversion of light produced by mercury lamps, by organic light-emitting diodes (OLEDs) or by white LEDs.

Particularly, they are suitable for conversion of light generated by a blue LED which offer high color rendering index CRI Ra and high R9 value.

In a further aspect, the invention relates to the use of a color converter as defined above for conversion of light generated by a cool white LED having a correlated color temperature between 3 000 K and 20 000 K to provide white light having a lower correlated color temperature and a high CRI Ra of greater than 90, preferably at least 92 and in particular more than 95. More particularly, they are suitable for conversion of light generated by white LEDs with a CCT between 20 000 K to 3 000 K, such as 20 000 K to 8 000 K, 20 000 to 6 000 K, 15 000 K to 8 100 K, 12 000 K to 8200 K, 10 000 to 6 000 K or 10 000 to 4 000 K to generate light having a lower CCT, whose CRI Ra is greater than 90, preferably at least 92 and in particular more than 95. In other words, the inventive color converters are capable to shift the wavelength of the white light source towards longer wavelength direction (i.e. redshift) to generate white light with a warm light tone and high color quality, such as high CRI Ra and R9.

White LEDs with a CCT beween 3 000 K to 20 000 K are commercially available. Blue LEDs with a center wavelength of emission between 400 nm and 480 nm are also commercially available.

The inventive color converters are additionally suitable for applications as a light-collecting system (fluorescence collector) in photovoltaics and in fluorescence conversion solar cells.

Inventive fluorescent colorants of formula (I) have a high photostability on illumination with light generated by blue LEDs with a center wavelength of emission between 420 nm and 480 nm. Inventive fluorescent colorants of formula (I) have also a high photostability on illumination with light generated by white LEDs with a CCT between 20 000 K to 6 000 K. Moreover, inventive fluorescent colorants of formula (I) are stable toward oxygen and water. They can be easily obtained in pure form.

Inventive color converters can be produced by different processes.

In one embodiment, the process for producing inventive color converters comprise the dissolution of the at least one polymer and the at least one organic fluorescent colorant and, if present, further organic fluorescent colorants, in a solvent and subsequent removal of the solvent.

In another embodiment, the process for producing inventive color converters comprise the extrusion of the at least one organic fluorescent colorant and, if present, further organic fluorescent colorants with the at least one polymer.

A light emitting device comprising at least one compound of formula (I), at least one orange fluorescent colorant having a maximum of emission in the wavelength range of 540 to below 600 nm and at least one red fluorescent colorant having a maximum of emission in the wavelength range of 600 to 670 nm leads to excellent properties in terms of CCT and CRI Ra. Thus, a further aspect of the invention relates to lighting devices comprising at least one white LED having a CCT between 3 000 K and 20 000 K and at least one color converter as defined above, wherein the color converter and the LED are in remote phosphor arrangement.

Still a further aspect of the invention relates to lighting devices comprising at least one blue LED with a center wavelength of emission between 400 nm and 480 nm and at least one color converter as defined above, wherein the color converter and the LED are in remote phosphor arrangement.

In one embodiment, inventive lighting devices comprise exactly one LED. In another embodiment, inventive lighting devices comprise two or more LEDs. Especially, inventive lighting devices comprise a plurality of LEDs

In one embodiment, inventive lighting devices comprise a plurality of LEDs, all of which are blue ones. In another embodiment, inventive lighting devices comprise a plurality of LEDs, at least one LED being blue and at least one LED not being a blue one but emitting light in another color, for example red.

Furthermore, the type of LED used is not crucial for the inventive lighting devices. In a preferred embodiment, the power density of the blue LED light impinging the surface of the converter plate is usually less than 200 mW/cm², preferably less than 120 mW/cm², more preferably less than 80 mW/cm². The use of LEDs of higher power densities, such as 150 or 200 mW/cm², is likewise possible. However, a higher power density of the LED at the converter surface can reduce the lifetime of the fluorescent colorants and the color converters.

In a further embodiment, inventive lighting devices comprise a plurality of LEDs selected from a blue LED with a center wavelength of emission between 400 nm and 480 nm and a white LED having a CCT between 3 000 K and 20 000 K.

Inventive color converters can be used in combination with LEDs selected from blue LEDs with a center wavelength of emission between 400 nm and 480 nm and white LEDs having a CCT between 3 000 K and 20 000 K in virtually any geometric form and irrespective of the construction of the lighting device.

Inventive color converters are used in a remote phosphor setup, i.e.., the color converter is spatially separated from the LED. In general, the distance between LED and color converter is from 0.1 cm to 50 cm, preferably 0.2 to 10 cm and most preferably 0.5 to 3 cm. Between color converter and LED may be different media such as air, noble gases, nitrogen or other gases or mixtures thereof.

The color converter may, for example, be arranged concentrically around the LED or have a planar geometry. It may take the form, for example, of a plaque, sheet or film, be in droplet form or take the form of a casting.

Inventive lighting devices are suitable for lighting in interiors, outdoors, of offices, of vehicles, in torches, games consoles, streetlights, traffic signs.

Inventive lighting devices exhibit warm-tone white light with a high average color rendering index. In addition, they have a long lifetime, especially a high photostability on illumination with blue light.

The present invention further provides a device producing electric power upon illumination comprising a photovoltaic cell (solar cell) and the color converter as defined above, where at least a part of the light not absorbed by the photovoltaic cell (solar cell) is absorbed by the color converter. The color converter is usually on top of the photovoltaic cell. The color converter is used to modify the spectrum such that UV and visible light are converted to a more bathochromic spectrum that is converted at higher efficiency by the solar cell.

The present invention is now illustrated in further detail by the following examples, without imposing any limitation thereto.

### Examples

### I. Preparation of compound of formula (I)

### Example 1: Mixture of compounds of formulae (I-A.1) and (I-A.2)

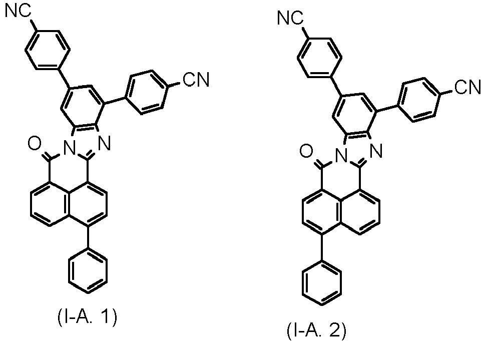

### 1.1 2,4-Dibromo-6-nitroaniline

A mixture of 10 g (0.072 mol) of 2-nitroaniline, 100 mL of glacial acid, 14.5 mL (0.29 mol; 46.4 g) of bromine were heated at about 45°C. After 2 hours, further 3.0 mL (0.06 mol) of bromine were added and the reaction mixture was stirred for two further hours. Excess bromine was outgassed. To the reaction mixture water was added. The precipitate was sucked off, washed with water and dried to give 21.0 g (98%) of a yellow solid.
R_{f}(toluene/ etyl acetate 10:1) = 0.8.

### 1.2 4-[4-Amino-3-(4-cyanophenyl)-5-nitro-phenyl]benzonitrile

A mixture of 367 mL of toluene, 19.45 g (0.066 mol) of the compound of example 1.1, 21.72 g (0.242 mol) of 4-cyanophenylboronic acid, 31.6 g (0.114 mol) of potassium carbonate dissolved in 50 mL of water, 6.02g (0.0066 mol) of tris(dibenzylideneacetone)dipalladium and 26 mL (0.0264 mol) of a tri-tert-butylphosphine solution in toluene were heated under nitrogen at 80 to 90°C for 3 hours. The reaction mixture was cooled to room temperature. The precipitate was filtered, washed with water and dried to 21.6 g (96%) of a yellow solid. R_{f}(toluene/ethyl acetate 10:1) = 0.29.

### 1.3 4-[3,4-Diamino-5-(4-cyanophenyl)phenyl]benzonitrile

A mixture of 19.9 g (0.0584 mol) of the compound of example 1.2, 400 mL of ethanol, 100 mL of N-methylpyrrolidone and 44.0 g (0.2328 mol) of zinc(II) chloride were heated under reflux at 85°C for 2 hours. After cooling to room temperature and filtration, ethanol was removed from the filtrate by distillation. The title compound was precipitated by addition of water and ethanol. The precipitate was filtered off, washed with hot water and dried in vacuum to give 25.9 g (143%) of a yellow compound containing inorganic salts.
R_{f}(toluene/ ethyl acetate 10:1) = 0.1.

### 1.4 Mixture of

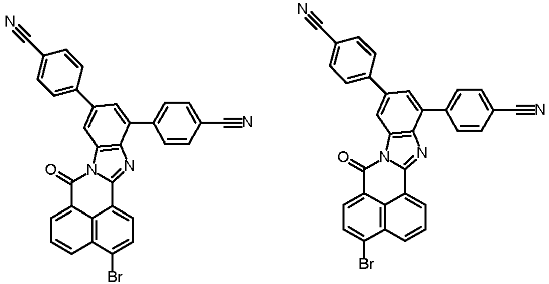

A mixture of 250 mL of quinoline, 8.8 g (0.032 mol) of 4-bromo-1,8-naphthalic anhydride, 11.0 g (0.032 mol; 90% purity) of the mixture from example 1.3, 6.0 g (0.032 mol) of zinc acetate was heated at 130°C for 2 hours under nitrogen. After cooling to room temperature, 200 mL of methanol were added. The mixture was stirred over night followed by filtration. The residue was washed with methanol and water. 11.45 g (65%) of a yellow precipitate were obtained.
R_{f}(toluene/ethyl acetate 10:1) = 0.5.

### 1.5 Mixture of compound of formulae (I-A.1) and (I-A.2)

A mixture of 11.0 g (0.02 mol) of the mixture of compounds from example 1.4, 2.68 g (0.02 mol) of phenylboronic acid, 5.52 g (0.04 mol) of potassium carbonate, 30 mL of water, 250 mL of toluene and 0.23 g (0.0002 mol) of tetrakistriphenylphosphinepalladium was heated at 90°C for 2 hours. After cooling to room temperature, the residue was filtered off, washed with methanol and water and dried in vacuum to give 10.5 (95%) of a yellow-black residue. This residue was dissolved in 400 mL of toluene by heating under reflux, 2.0 g of activated charcoal were added, the mixture was stirred for 30 minutes followed by hot filtration. The filtrate was allowed to cool up over night and the precipitate was filtered off. Yield: 2.3 g of the title compound which is free of palladium.
R_{f}(toluene/ethyl acetate 10:1) = 0.5.
Lambda max emission: 519 nm (in polycarbonate).

### Example 2: Compound of formula (I-A.3)

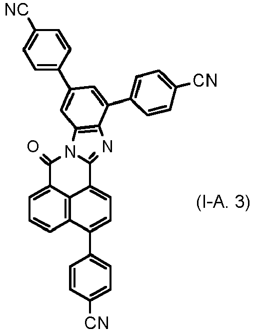

### 2.1 Preparation of

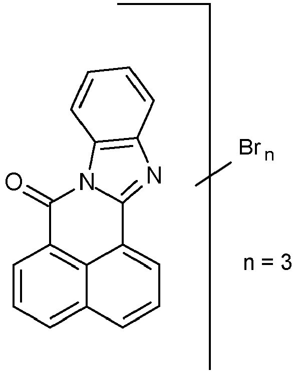

Following the procedure described in example 6 of WO 2012/168395, a mixture of the title compound and of the corresponding mono- and di- and tetrabrominated compounds were obtained. The tribrominated compound constitutes about 40% by weight.

### 2.2 Preparation of compound (I-A.3)

A mixture of 2.5 g (0.005 mol) g of the tribrominated compound of example 2.1, 4.41 g (0.03 mol) of 4-cyanophenylboronic acid, 2.07 g (0.015 mol) of potassium carbonate dissolved in 5 mL of water and 0.174 g (0.00015 mol) of tetrakistriphenylphosphinepalladium(0) was heated at 90°C for 4 hours. After cooling to room temperature, the residue was filtered off, washed with methanol and water and dried in vacuum to give 2.29 g of a crude product. The compound was purified by column chromatography (silica gel; toluene/ethyl acetate 20:1) to afford 0.92 g (32%) of a yellow solid.
R_{f}(toluene/ethyl acetate 10:1) = 0.3.
Lambda max emission = 508 nm (polycarbonate).

### II. Preparation of color converters

### Materials used:

LED 1: cool white LED with CCT of 8595 K
LED 2: blue LED with maximum wavelength of 450 nm

Polymer 1: transparent polycarbonate based on a polycondensate of bisphenol A and phosgene (Makrolon® 2805 from Bayer MaterialScience AG). In the examples, polymer 1 was used, although comparable data could be achieved with other polymers.

Titanium dioxide: TiO₂ rutile pigment: Kronos® 2233 -from Kronos Titan

### Colorant 1: Yellow fluorescent compound (not according to the invention)

Compound of formula (VI-5), in the following Col 1 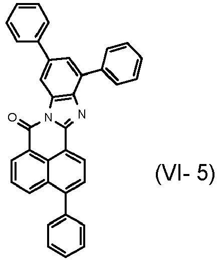 obtained as described in example 10 of WO 2012/168395, followed by purification with chromatography. The mixture comprising the compound VI-5 was subjected to a further column chromatography to give the pure compound VI-5.
Lambda max emission: 536 nm (in polycarbonate).

### Colorant 2: Mixture of inventive compounds of formulae (I-A.1) and (I.-A.2) (Compounds from example 1), in the following Col 2

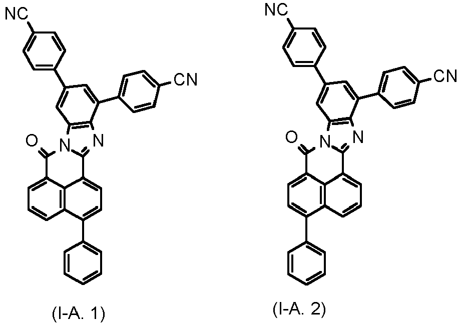

Lambda max emission: 519 nm (in polycarbonate).

### Colorant 3: Inventive compound of formula (I-A.3)

(compound from example 2), in the following Col 3

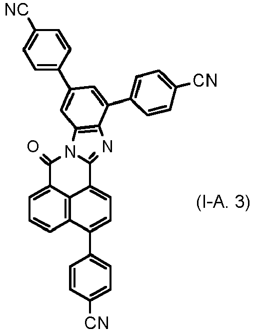

Lambda max emission: 508 nm (in polycarbonate)

### Colorant 4: Compound of formula (III.1), in the following Col 4

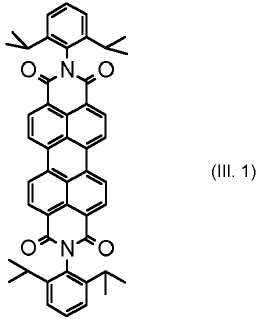

Lambda max emission: 548 and 578 nm (in polycarbonate)

### Colorant 5: Compound of formula (II.1), in the following Col 5

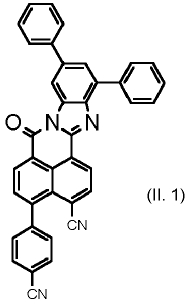

In analogy to the methods described in WO 2015/019270, the compound of formula (II.1) was prepared as follows:

### Step 1: Preparation of

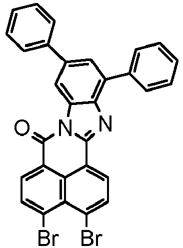

A mixture of 42.72 g (0.12 mol) of 4,5-dibromo-nahthalene-1,8-dicarboxylic acid anhydride, 42.96 g of (0.12 mol) 1,2 diamino-3,5-diphenylbenzene, 500 ml of quinoline, 22.0 g (0.06mol) of zinc acetate was heated 135°C for 4 hours. The reaction mixture was cooled to 70°C and the product precipitated upon addition of 500 mL of ethanol. The product was filtered off, washed with ethanol and water. 58.1 g of product was isolated.
Rf (toluene ethylacetat 10:1) = 0.82.

### Step 2: Preparation of

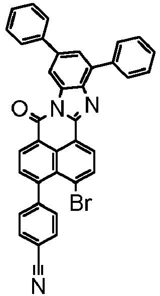

A mixture of 88 mL of toluene, 3.3 g (5.5 mmol) of the above mentioned compound from step 1, 2.2 g (16 mmol) of potassium carbonate dissolved in 20 mL of water, 0.8 g (5.5 mmol) of 4-cyanophenylboronic acid, 0.64 g (0.55 mmol) of tetrakistriphenylphosphinepalladium was stirred at 100°C for 18 hours. Another 0.64 g (0.55 mmol) of tetrakistriphenylphosphinepalladium were added and the mixture was stirred for another 2 hours. 0.4 g (2.35 mmol) of 4-cyanophenylboronic acid were added and the mixture was stirred for another 8 hours. The product was precipitated with 500 mL of methanol, filtered and washed with water and methanol and dried under reduced pressure. The product was purified by column chromatography on silica using toluene as eluent. 0.7 g of pure product were isolated. Rf (toluene) = 0.2.

### Step 3: Preparation of the title compound of formula (II.1)

A mixture of 10 mL of N-methylpyrrolidone, 0.7 g (1.2 mmol) of the above mentioned compound from step 2, 135 mg (1.5 mmol) of CuCN was heated to 160°C for 2 hours. Another 135 mg (1.5 mmol) CuCN were added and the mixture was stirred for one additional hour. The reaction mixture was cooled to room temperature and 10 mL of methanol and 10 mL of water were added. After stirring for one hour the mixture was filtered, washed with methanol and water, dried under reduced pressure and the crude material was purified by column chromatography. A column with a diameter of 6.5 cm was filled with 120 g of silica to reach a height of 6 cm. A mixture of toluene ethylacetate 100:1 was used. The product was isolated with an Rf value of 0.37. Chromatography was repeated under identical conditions and 8 mg of pure product crystallized from the toluene ethy lacetate mixture upon standing overnight.
Lambda max emission: 568 nm (in polycarbonate).

### Colorant 6: Compound of formula (IV.1), in the following Col 6

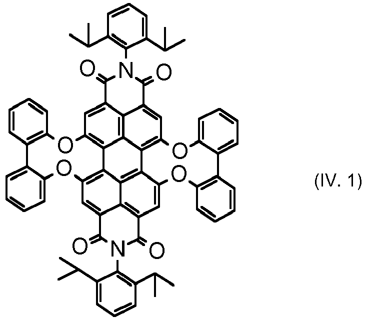

prepared as described in example 1 of PCT/EP2017/050621.

A mixture of 15 g (17.7 mmol) N,N'-(2,6-diisopropylphenyl)-1,6,7,12,-tetrachloroperylene-3,4;9,10 diimide, 6.9 g (37.1 mmol) of 2,2'-biphenol, 5.13 g (37.1 mmol) of K₂CO₃ and 90 mL of N-Methyl-2-pyrrolidone (NMP) were heated at 110°C for 21 h and then at 140°C for 24 h. The reaction mixture was allowed to cool to 80°C followed by adding 90 mL of acetic acid/water (1/2) within 60 min under stirring (125 rpm) and subsequently stirring over night. The residue was filtered off and washed with a mixture of 90 mL of ethanol and 20 mL of NMP and then with warm water. The residue was dried to give a crude product (16.81 g). Purification by chromatography affords the title compound.
R_{f} (cyclohexane/ethyl acetate 10:1) = 0.1.
Lambda max emission: 579 nm (in polycarbonate).

### Colorant 7: Compound of formula (V.2), in the following Col 7

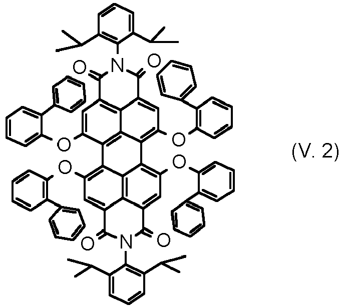

prepared as described in example 1 of EP 16192617.5.

A mixture of 5 g (5.9 mmol) of N,N'-(2,6-diisopropylphenyl)-1,6,7,12,-tetrachloroperylenetetracarboxylic diimide, 4.23 g of (24.9 mmol) biphenyl-2-ol, 138.21 g (16.9 mmol) of potassium carbonate and 30 mL of N-methyl-2-pyrrolidone (NMP) were stirred at room temperature for 24 h and then for 48 h at 115°C. After cooling to 80°C the reaction mixture was added tropwise to a mixture of 10 mL of acetic acid and 20 mL of water within 15 min, cooled to room temperature over a period of 2 h and then filtered. The residue was washed with 300 mL of a mixture of ethanol/ water (1:1) and then with 600 mL of a mixture of ethanol/water/NMP (4:4:1). The residue was dissolved in a mixture of 35 mL of ethanol and 5 mL of NMP under reflux, then cooled to room temperature and separated to obtain 5.6 g (62%) of a red colorant which was purified by chromatography using cyclohexane/ethyl acetate. The yield was 2.06 g (23%).
Rf (cylohexane/ethyl acetate 10:1) = 0.29.
Lambda max emission: 622 nm (in polycarbonate).

### Colorant 8: Compound of formula (V.3), in the following Col 8

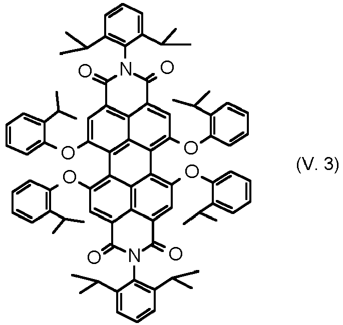

A mixture of 2.2.g (2.6 mmol) of 1,6,7,12-tetrachloro-N,N'-2,6-diisopropylphenylperylene-3,4,9,10-tetracarboxylic acid diimide, 4.25 g (31.2 mmol) of 2-isopropylphenol, 2.52 g (18.2 mmol) of K₂CO₃ and 170 mL of N-methylpyrrolidone were heated to 90°C for 17 hours. Afterwards the mixture was heated to 110°C for 10 hours. Further 2.12 g (15.6 mmol) of 2-isopropylphenol and 1.26 g of K₂CO₃ were added and heating continued for 23 hours. Further 2.12 g (15.6 mmol) of 2-isopropylphenol and 1.26 g of K₂CO₃ were added and heating continued for 6 hours. The product was precipitated with 1L of diluted HCl. After extraction with dichloromethane 7.5 g of a liquid crude material was obtained which was further purified by column chromatography using toluene dicholoromethane. 0.28 g of pure title compound were isolated.
Rf (petroleum ether / ethylacetate 8:1) = 0.3.
Lambda max emission: 616 nm (in polycarbonate)

### Colorant 9: Compound of formula (V.1), namely N,N'-bis(2,6-diisopropylphenyl)-1,6,7,12-tetraphenoxyperylene-3,4;9,10-tetracarboxylic acid diimide, in the following Col 9

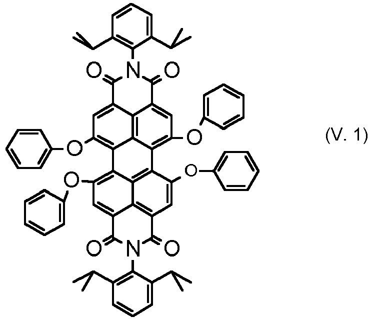

Lambda max emission: 615 nm (in polycarbonate)

### Fluorescence spectra

Fluorescence quantum yields (FQY) of the analysis samples were measured with the C9920-02 quantum yield measuring system (from Hamamatsu). This was done by illuminating each of the samples with light of 445 to 455 nm in an integration sphere (Ulbricht sphere). By comparison with the reference measurement in the Ulbricht sphere without sample, the unabsorbed fraction of the excitation light and the fluorescent light emitted by the sample are determined by means of a CCD spectrometer. Integration of the intensities over the spectrum of the unabsorbed excitation light or over that of the emitted fluorescent light gives the degree of absorption or fluorescence intensity or fluorescence quantum yield of each sample.
PC = polycarbonate

### Results of fluorescent quantum yield measurements:

### Col 1: (not according to the invention)

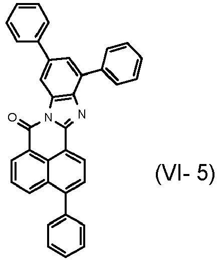

PC-film (0.01 % by weight): Emission λₘₐₓ : 536 nm; FQY: 86%.

### Col 2 (according to the invention)

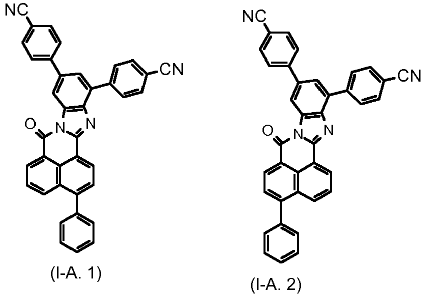

PC-film (0.022% by weight): Emission λₘₐₓ : 519 nm; FQY: 87%.

### Col 5 (not according to the invention)

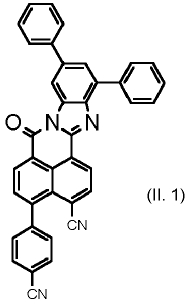

PC-film (0.014% by weight): Emission λₘₐₓ : 568 nm; FQY: 85%.

As can be seen, lambda max emission of Col 2 according to the invention is shifted to a shorter wavelength in comparison to a compound (Col 1) carrying phenyl groups instead of cyanophenyl groups at the benzimidazole skeleton or a compound (Col 5) carrying a cyano group at the core of the naphthoylene skeleton (Col 5). Accordingly, Col 2 can close the gap in the green spectral range in contrast to the structurally related prior art compounds Col 1 and Col 5.

### Method for production of color converters:

For preparation of the converters, the materials, i.e. polycarbonate, colorants and TiO₂ (Kronos 2233) were mixed together according to the desired concentrations (see Table II and III). The amounts of materials are given relative to the amount of polymer polycarbonate used. Then methylene chloride was added and the mixtures were stirred overnight. The solutions/dispersions obtained were coated onto a glass surface using an applicator frame (wet film thickness 800 µm from Ericsen). After the solvent had dried off for 2 hours, the film was detached from the glass and dried in vacuum at 50°C. From these films, samples with different geometries were cut from the foils, depending on application (with LED1 or LED2 resp.).

**Table II: Non-inventive converter mixtures with Col 1 for cool-white LEDs**

| Example | Yellow colorant | Yellow conc.^{#} | Orange colorant | Orange conc.^{#} | Red colorant | Red conc.^{#} | TiO₂ [% by weight] | Film thickness [µm] |
|---|---|---|---|---|---|---|---|---|
| C1 | Col 1 | 0.03405 | Col. 4 | 0.00176 | Col 7 | 0.00881 | 0.50 | 152.6 |
| C2 | Col 1 | 0.0289 | Col. 4 | 0.00133 | Col 8 | 0.00667 | 0.50 | 150.6 |

| | | | | | | | | |
|---|---|---|---|---|---|---|---|---|
| ^{#} concentration in % by weight based on the amount of polymer polycarbonate used | | | | | | | | |

**Table III: Inventive converter mixtures with yellow col 2 and col 3 and orange col 5 for cool-white LEDs**

| Example | Yellow colorant | Yellow conc.^{#} | Orange colorant | Orange conc.^{#} | Red colorant | Red conc.^{#} | TiO₂ [% by weight.] | Film thickness [µm] |
|---|---|---|---|---|---|---|---|---|
| A1 | Col 2 | 0.0399 | Col 4 | 0.0020 | Col 7 | 0.010 | 0.50 | 155.3 |
| A2 | Col 2 | 0.0390 | Col 4 | 0.0016 | Col 8 | 0.0080 | 0.50 | 153.6 |
| A3 | Col 2 | 0.0414 | Col 5 | 0.0028 | Col 7 | 0.0113 | 0.50 | 148.3 |
| A4 | Col 2 | 0.0444 | Col 6 | 0.00206 | Col 7 | 0.0103 | 0.50 | 148.0 |
| A5 | Col 2 | 0.0432 | Col 6 | 0.00157 | Col 8 | 0.00783 | 0.50 | 150.0 |
| A6 | Col 3 | 0.0827 | Col 4 | 0.00205 | Col 7 | 0.01023 | 0.50 | 153.0 |

| | | | | | | | | |
|---|---|---|---|---|---|---|---|---|
| ^{#} concentration in % by weight based on the amount of polymer polycarbonate | | | | | | | | |

### Characterization of the lighting devices:

LED 1 and LED 2, respectively, were used as light source for pumping the converter film.

The cool-white LEDs 1 were inserted into a transparent plastic tube of T8 format. Rectangular pieces of the converter films are shaped to semitubes and inserted into the tube. The converter film therefore covers the cool white LEDs.

LED 2: A down-light equipped with blue LEDs (450 nm) inside a mixing chamber are totally covered by a planar, circular platelet of the converter film with 61 mm diameter.

The light irradiated from the surface of these devices was subjected to the photometric measurement, where the total light irradiated from the device was measured by a photometric measurement tool equipped with an integrating sphere, ISP 500-100, and the CCD detector CAS 140CT-156 (from Instrument Systems, Munich). The measured radiance spectrum was used to derive all relevant photometric data such as CCT (= correlated color temperature) in Kelvin [K], distance of color point from Planck-curve (BBL), average color rendering index CRI and color rendering index for reference color no. 9 (R9), efficacy data etc. The results are given in tables III and IV and VI.

**Table IV: Photometric data of non-inventive converters for cool white LED 1 (for comparison)**

| | CIE-x | CIE-y | CIE-u' | CCT [K] | distance from BBL (duv) | average CRI (Rₐ) | R9 |
|---|---|---|---|---|---|---|---|
| LED 1 | 0.2987 | 0.2766 | 0.2088 | 8595 | -1,80•10⁻² | 81.53 | 51.56 |
| C1 | 0.4290 | 0.4020 | 0.2463 | 3122 | 3.27•10⁻⁴ | 89.27 | 59.12 |
| C2 | 0.4332 | 0.4016 | 0.2492 | 3044 | -4.62•10⁻⁴ | 88.79 | 43.33 |

**Table V: Photometric data of inventive converters for cool white LED 1**

| | CIE-x | CIE-y | CIE-u' | CCT [K] | distance from BBL duv | average CRI Rₐ | R9 |
|---|---|---|---|---|---|---|---|
| LED 1 | 0.2987 | 0.2766 | 0.2088 | 8595 | -1,80•10⁻² | 81.53 | 51.56 |
| A1 | 0.4291 | 0.4004 | 0.2471 | 3106 | -3.28•10⁻⁴ | 95.28 | 78.6 |
| A2 | 0.4292 | 0.4002 | 0.2472 | 3103 | -4.46•10⁻⁴ | 95.13 | 67.5 |
| A3 | 0.4265 | 0.4034 | 0.2441 | 3179 | 1.32•10⁻³ | 94.87 | 88.9 |
| A4 | 0.4276 | 0.3997 | 0.2464 | 3128 | -3.68•10⁻⁴ | 95.16 | 85.2 |
| A5 | 0.4283 | 0.4006 | 0.2465 | 3123 | 1.25•10⁻⁴ | 94.56 | 70.6 |
| A6 | 0.4342 | 0.3996 | 0.2508 | 3009 | -1.42•10⁻³ | 95.87 | 77.0 |

**Table VI: Converters for blue LED**

| Example | Yellow colorant | Yellow conc.^{#} | Orange colorant | Orange conc.^{#} | Red colorant | Red conc.^{#} | TiO₂ [% by weight] | Film thickness [µm] |
|---|---|---|---|---|---|---|---|---|
| C3 | Col 1 | 0.123 | Col 4 | 0.0039 | Col 7 | 0.00995 | 0.50 | 148.1 |
| A7 | Col 2 | 0.150 | Col 4 | 0.0045 | Col 7 | 0.0116 | 0.50 | 144.2 |

**Table VII: Photometric data of converters for blue LED**

| | CIE-x | CIE-y | CIE-u' | CCT [K] | distance from BBL (duv) | average CRI (Rₐ) | R9 |
|---|---|---|---|---|---|---|---|
| LED 2 | 0.1539 | 0.0235 | 0.2069 | | -- | -- | -- |
| C3 | 0.4257 | 0.3903 | 0.2492 | 3083 | -4.01•10⁻³ | 91.78 | 65.57 |
| A7 | 0.4040 | 0.3945 | 0.2334 | 3558 | 1.93•10⁻³ | 94.75 | 95.0 |

Non-inventive and inventive converters are described in Tables II, III and VI. The photometric data are given in Tables IV and V and VII. The converters create warm- white light having almost similar CIE color coordinates. The inventive color converters exhibit significantly higher values of the average CRI and of R9. Since the orange and red colorants are the same in the comparative examples as in the inventive examples, the improvement of average CRI and R9 can be attributed to the inventive yellow colorant 2 and the inventive yellow colorant 3.

## Claims

1. A naphthoylenebenzimidazole compound of formula (I) 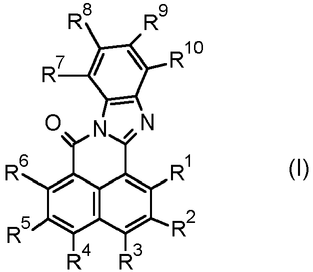 wherein
at least one of the radicals R¹, R², R³, R⁴, R⁵, R⁶, R⁷, R⁸, R⁹ and R¹⁰ independently of each other is aryl which carries one, two or three cyano groups and 0, 1, 2, 3 or 4 substituents R^{Ar} and the remaining radicals R¹, R², R³, R⁴, R⁵, R⁶, R⁷, R⁸, R⁹ and R¹⁰, independently of each other, are selected from hydrogen and aryl which is unsubstituted or carries 1, 2, 3, 4 or 5 substituents R^{Ar}, and wherein at least one of the radicals R⁷, R⁸, R⁹ and R¹⁰ is aryl which carries 1, 2 or 3 cyano groups and 0, 1, 2, 3 or 4 substituents R^{Ar} and the remaining radicals R⁷, R⁸, R⁹ and R¹⁰ are, independently of each other, selected from the group consisting of hydrogen and aryl which is unsubstituted or carries 1, 2 or 3 radicals R^{Ar};
where
R^{Ar} independently of each other and independently of each occurrence, is selected from halogen,
C₁-C₃₀-alkyl, C₂-C₃₀-alkenyl, C₂-C₃₀-alkynyl, where the three latter radicals are unsubstituted or carry one or more R^{a} groups, C₃-C₈-cycloalkyl, 3- to 8-membered heterocyclyl, where the two latter radicals are unsubstituted or carry one or more R^{b} groups, aryl and heteroaryl, where the two latter radicals are unsubstituted or carry one or more R^{c} groups,
where
R^{a}, independently of each other and independently of each occurrence, is selected from cyano, halogen, C₃-C₈-cycloalkyl, 3- to 8-membered heterocyclyl, aryl and heteroaryl, where C₃-C₈-cycloalkyl, 3- to 8-membered heterocyclyl are unsubstituted or bear one or more R^{b1} groups, and where aryl and heteroaryl are unsubstituted or bear one or more R^{c1} groups;
R^{b}, independently of each other and independently of each occurrence, is selected from cyano, halogen, C₁-C₁₈-alkyl, C₃-C₈-cycloalkyl, 3- to 8-membered heterocyclyl, aryl and heteroaryl, where C₃-C₈-cycloalkyl, 3- to 8-membered heterocyclyl are unsubstituted or bear one or more R^{b1} groups, and where aryl and heteroaryl are unsubstituted or bear one or more R^{c1} groups;
R^{c}, independently of each other and independently of each occurrence, is selected from cyano, halogen, C₁-C₁₈-alkyl, C₃-C₈-cycloalkyl, 3- to 8-membered heterocyclyl, aryl and heteroaryl, where C₃-C₈-cycloalkyl, 3- to 8-membered heterocyclyl are unsubstituted or bear one or more R^{b1} groups, and where aryl and heteroaryl are unsubstituted or bear one or more R^{c1} groups;
R^{b1}, independently of each other and independently of each occurrence, is selected from halogen, C₁-C₁₈-alkyl and C₁-C₁₈-haloalkyl,
R^{c1}, independently of each other and independently of each occurrence, is selected from halogen, C₁-C₁₈-alkyl and C₁-C₁₈-haloalkyl;
or a mixture thereof.

2. The compound of formula (I) as claimed in claim 1, wherein R^{Ar} is C₁-C₁₀-alkyl, C₁-C₆-haloalkyl, C₃-C₈-cycloalkyl or phenyl which is unsubstituted or carries 1, 2 or 3 radicals R^{c}.

3. The compound of formula (I) as claimed in claim 2, wherein 1, 2, 3 or 4 of the radicals R¹, R², R³, R⁴, R⁵, R⁶, R⁷, R⁸, R⁹ and R¹⁰ is/are phenyl which carries 1 or 2 cyano groups, wherein at least one of the radicals R⁷, R⁸, R⁹ and R¹⁰ is phenyl which carries 1 or 2 cyano groups; 0, 1, 2 or 3 of the radicals R¹, R², R³, R⁴, R⁵, R⁶, R⁷, R⁸, R⁹ and R¹⁰ are phenyl which is unsubstituted or carries 1, 2 or 3 radicals R^{Ar}; and the remaining radicals R¹, R², R³, R⁴, R⁵, R⁶, R⁷, R⁸, R⁹ and R¹⁰ are hydrogen.

4. The compound of formula (I) according to any of the preceding claims, which corresponds to a compound of formula (I-A) 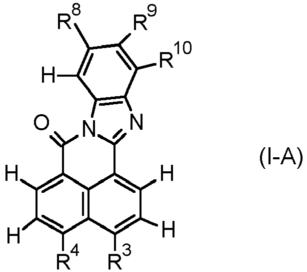 wherein
R³ and R⁴ are each independently hydrogen, phenyl which carries 1 or 2 cyano groups or phenyl which is unsubstituted or carries 1, 2 or 3 substituents selected from C₁-C₁₀-alkyl; and
at least one of the radicals R⁸, R⁹ and R¹⁰ is phenyl which carries 1 or 2 cyano groups; and the remaining radicals R⁸, R⁹ and R¹⁰ are selected from the group consisting of hydrogen and phenyl which is unsubstituted or carries 1, 2 or 3 substituents selected from C₁-C₁₀-alkyl.

5. The use of a compound of the formula (I) as defined in any of claims 1 to 4 or a mixture thereof in color converters for converting light emitted from a blue LED with a center wavelength of emission between 400 nm and 480 nm into light of a second, longer wavelength or for converting light emitted from a white LED having a correlated color temperature between 3 000 K and 20 000 K to provide white light having a lower correlated color temperature.

6. A color converter comprising in a polymeric matrix
- at least one naphthoylenebenzimidazole compound of the formula (I) as defined in any of claims 1 to 4 as fluorescent colorant and at least one further organic fluorescent colorant which is selected from
(a) an organic orange fluorescent colorant having a maximum of emission in the wavelength range of 540 to less than 600 nm and mixtures thereof; and
(b) an organic red fluorescent colorant having a maximum of emission in the wavelength range of 600 to 670 nm and mixtures thereof; and mixtures thereof;
- and optionally at least one inorganic white pigment as a scattering body.

7. The color converter as claimed in claim 6, wherein the organic orange fluorescent colorant having a maximum of emission in the wavelength range of 540 to less than 600 nm is selected from
(a.1) a core-cyanated naphthoyalenebenzimidazole compound of the formula (II) 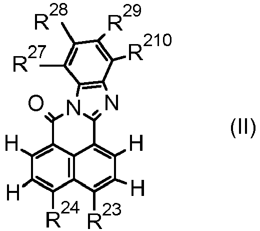 wherein
one of R²³ or R²⁴ independently of each other is cyano and the other radical R²³ or R²⁴ is selected from cyano, phenyl, 4-cyanophenyl and phenyl which carries 1, 2 or 3 substituents selected from C₁-C₁₀-alkyl;
R²⁷, R²⁸, R²⁹ and R²¹⁰ independently of each other are hydrogen, phenyl, 4-cyanophenyl or phenyl which carries 1, 2 or 3 substituents selected from C₁-C₁₀-alkyl,
and mixtures thereof.
(a.2) a perylene bisimide compound of the formula (III) 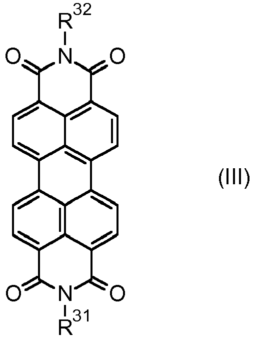 wherein
R³¹ and R³² independently of each other are C₁-C₃₀-alkyl, C₃-C₈-cycloalkyl, aryl, heteroaryl or aryl-C₁-C₁₀-alkylene, where the (hetero)aromatic ring in the three latter radicals is unsubstituted or mono- or polysubstituted by C₁-C₁₀-alkyl;
(a.3) a perylene bisimide compound with rigid 2,2'-biphenoxy bridges of the formula (IV) 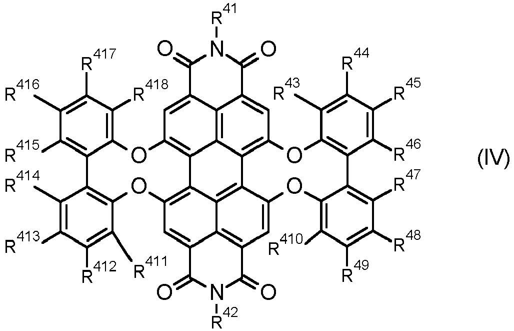 wherein
R⁴¹ and R⁴², independently of each other, are selected from hydrogen, C₁-C₁₀-alkyl, which is unsubstituted or substituted by C₆-C₁₀-aryl which in turn is unsubstituted or substituted by 1, 2 or 3 C₁-C₁₀-alkyl, C₃-C₈-cycloalkyl, which is unsubstituted or substituted by 1, 2 or 3 C₁-C₁₀-alkyl, and C₆-C₁₀-aryl which is unsubstituted or substituted by 1, 2 or 3 C₁-C₁₀-alkyl;
R⁴³, R⁴⁴, R⁴⁵, R⁴⁶, R⁴⁷, R⁴⁸, R⁴⁹, R⁴¹⁰, R⁴¹¹, R⁴¹², R⁴¹³, R⁴¹⁴, R⁴¹⁵, R⁴¹⁶, R⁴¹⁷and R⁴¹⁸ independently of each other, are selected from hydrogen, halogen, C₁-C₁₈-alkyl, C₆-C₁₀-aryl, C₆-C₁₀-aryl-C₁-C₁₀-alkylene, C₆-C₁₀-aryloxy and C₆-C₁₀-arylthio, where the aryl moiety of C₆-C₁₀-aryl, C₆-C₁₀-aryl-C₁-C₁₀-alkylene, C₆-C₁₀-aryloxy and C₆-C₁₀-arylthio is unsubstituted or substituted by one or more C₁-C₁₀-alkyl,
and mixtures thereof.

8. The color converter as claimed in claim 7, wherein the organic orange fluorescent colorant having a maximum of emission in the wavelength range of 540 to less than 600 nm is selected from colorants of the formulae (II.1), (III.1) and (IV.1)

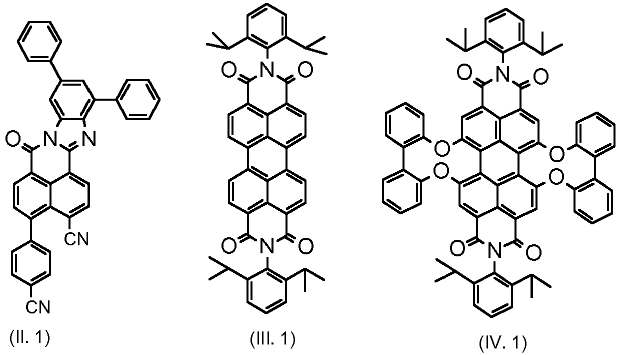

9. The color converter as claimed in any of claims 6 to 8, wherein the organic red fluorescent colorant having a maximum of emission in the wavelength range of 600 to 670 nm is a perylene bisimide compound of formula (V) 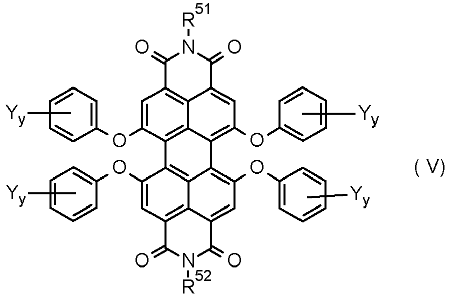 wherein
R⁵¹ and R⁵² independently of each other are C₁-C₁₀-alkyl, which is unsubstituted or substituted by C₆-C₁₀-aryl which in turn is unsubstituted or substituted by 1, 2 or 3 C₁-C₁₀-alkyl,
C₃-C₈-cycloalkyl, which is unsubstituted or substituted by 1, 2 or 3 C₁-C₁₀-alkyl, or
C₆-C₁₀-aryl which is unsubstituted or substituted by 1, 2 or 3 C₁-C₁₀-alkyl;
Y independently of each other and independently of each occurrence is C₁-C₁₀-alkyl, phenyl, or phenyl which is substituted by 1, 2 or 3 C₁-C₆-alkyl, C₁-C₆-alkoxy-C₁-C₆-alkyl and C₁-C₆-alkoxy; and
y independently of each other and independently of each occurrence is 0, 1, 2, or 3.

10. The color converter as claimed in claim 9, wherein the organic red fluorescent colorant having a maximum of emission in the wavelength range of 600 to 670 nm is selected from a perylene bisimide compound of formulae (V.1), (V.2), and (V.3)

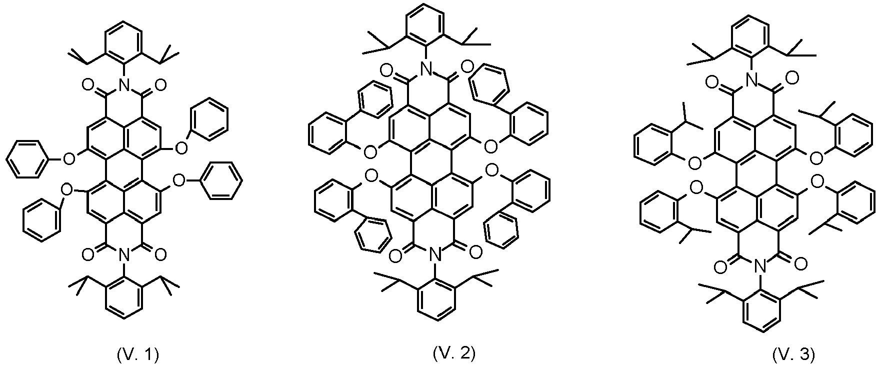

11. The color converter as claimed in any of claims 6 to 10, comprising a combination of organic fluorescent colorants selected from a compound of formulae (I-A) + (II.1) + (V.1), (I-A) + (II.1) + (V.2), (I-A) + (II.1) + (V.3), (I-A) + (III.1) + (V.1), (I-A) + (III.1) + (V.2), (I-A) + (III.1) + (V.3), (I-A) + (IV.1) + (V.1), (I-A) + (IV.1) + (V.2), and (I-A) + (IV.1) + (V.3).

12. The color converter as claimed in any of claims 6 to 11, wherein the polymeric matrix consists essentially of polystyrene, polycarbonate, polymethyl methacrylate, polyvinylpyrrolidone, polymethacrylate, polyvinyl acetate, polyvinyl chloride, polybutene, silicone, polyacrylate, epoxy resin, polyvinyl alcohol, poly(ethylene vinylalcohol)-copolymer, polyacrylonitrile, polyvinylidene chloride, polystyrene acrylonitrile (SAN), polybutylene terephthalate, polyethylene terephthalate, polyvinyl butyrate, polyvinyl chloride, polyamides, polyoxymethylenes, polyimides, polyetherimides or mixtures thereof, preferably polystyrene, polycarbonate, or polyethylene terephthalate.

13. The use of a color converter as defined in any of claims 6 to 12 for conversion of light generated by a blue LED with a center wavelength of emission between 400 nm and 480 nm to provide white light or for conversion of light generated by a white LED having a correlated color temperature between 3 000 K and 20 000 K to provide white light having a lower correlated color temperature.

14. A lighting device comprising
(i) at least one LED selected from a blue LED with a center wavelength of emission from 400 nm to 480 nm and a white LED having a correlated color temperature between 3 000 K and 20 000 K; and
(ii) at least one color converter as defined in any of claims 6 to 12,
wherein the at least one color converter is in a remote arrangement from the at least one LED.

15. A device producing electric power upon illumination comprising a photovoltaic cell and the color converter as defined in any of claims 6 to 12, where at least a part of the light not absorbed by the photovoltaic cell is absorbed by the color converter.

## Patentansprüche

1. Naphthoylenbenzimidazol-Verbindung der Formel (I) 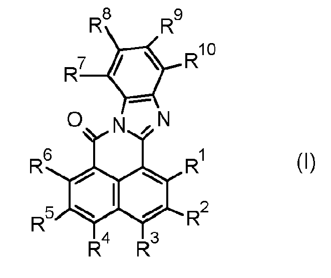 worin
mindestens einer der Reste R¹, R², R³, R⁴, R⁵, R⁶, R⁷, R⁸, R⁹ und R¹⁰ unabhängig voneinander für Aryl, das eine, zwei oder drei Cyanogruppen und 0, 1, 2, 3 oder 4 Substituenten R^{Ar} trägt, steht und die übrigen Reste R¹, R², R³, R⁴, R⁵, R⁶, R⁷, R⁸, R⁹ und R¹⁰ unabhängig voneinander aus Wasserstoff und Aryl, das unsubstituiert ist oder 1, 2, 3, 4 oder 5 Substituenten R^{Ar} trägt, ausgewählt sind und wobei mindestens einer der Reste R⁷, R⁸, R⁹ und R¹⁰ für Aryl, das 1, 2 oder 3 Cyanogruppen und 0, 1, 2, 3 oder 4 Substituenten R^{Ar} trägt, steht und die übrigen Reste R⁷, R⁸, R⁹ und R¹⁰ unabhängig voneinander aus der Gruppe bestehend aus Wasserstoff und Aryl, das durch 1, 2 oder 3 Reste R^{Ar} substituiert ist, stehen;
wobei
R^{Ar} unabhängig voneinander und unabhängig von jedem Vorkommen aus Halogen,
C₁-C₃₀-Alkyl, C₂-C₃₀-Alkenyl, C₂-C₃₀-Alkinyl, wobei die drei letztgenannten Reste unsubstituiert oder eine oder mehrere Gruppen R^{a} tragen,
C₃-C₈-Cycloalkyl, 3- bis 8-gliedrigem Heterocyclyl, wobei die zwei letztgenannten Reste unsubstituiert oder eine oder mehrere Gruppen R^{b} tragen,
Aryl und Heteroaryl, wobei die zwei letztgenannten Reste unsubstituiert oder eine oder mehrere Gruppen R^{c} tragen, ausgewählt ist,
wobei
R^{a} unabhängig voneinander und unabhängig von jedem Vorkommen aus Cyano, Halogen, C₃-C₈-Cycloalkyl, 3- bis 8-gliedrigem Heterocyclyl, Aryl und Heteroaryl ausgewählt ist, wobei C₃-C₈-Cycloalkyl und 3- bis 8-gliedriges Heterocyclyl unsubstituiert sind oder eine oder mehrere Gruppen R^{b1} tragen und wobei Aryl und Heteroaryl unsubstituiert sind oder eine oder mehrere Gruppen R^{c1} tragen;
R^{b} unabhängig voneinander und unabhängig von jedem Vorkommen aus Cyano, Halogen, C₁-C₈-Alkyl, C₃-C₈-Cycloalkyl, 3- bis 8-gliedrigem Heterocyclyl, Aryl und Heteroaryl ausgewählt ist, wobei C₃-C₈-Cycloalkyl und 3- bis 8-gliedriges Heterocyclyl unsubstituiert sind oder eine oder mehrere Gruppen R^{b1} tragen und wobei Aryl und Heteroaryl unsubstituiert sind oder eine oder mehrere Gruppen R^{c1} tragen;
R^{c} unabhängig voneinander und unabhängig von jedem Vorkommen aus Cyano, Halogen, C₁-C₈-Alkyl, C₃-C₈-Cycloalkyl, 3- bis 8-gliedrigem Heterocyclyl, Aryl und Heteroaryl ausgewählt ist, wobei C₃-C₈-Cycloalkyl und 3- bis 8-gliedriges Heterocyclyl unsubstituiert sind oder eine oder mehrere Gruppen R^{b1} tragen und wobei Aryl und Heteroaryl unsubstituiert sind oder eine oder mehrere Gruppen R^{c1} tragen;
R^{b1} unabhängig voneinander und unabhängig von jedem Vorkommen aus Halogen, C₁-C₁₈-Alkyl und C₁-C₁₈-Halogenalkyl aus-gewählt ist;
R^{c1} unabhängig voneinander und unabhängig von jedem Vorkommen aus Halogen, C₁-C₁₈-Alkyl und C₁-C₁₈-Halogenalkyl ausgewählt ist;
oder eine Mischung davon.

2. Verbindung der Formel (I) nach Anspruch 1, worin R^{Ar} für C₁-C₁₀-Alkyl, C₁-C₆-Halogenalkyl, C₃-C₈-Cycloalkyl oder Phenyl, das unsubstituiert oder 1, 2 oder 3 Reste R^{c} trägt, steht.

3. Verbindung der Formel (I) nach Anspruch 2, wobei 1, 2, 3 oder 4 der Reste R¹, R², R³, R⁴, R⁵, R⁶, R⁷, R⁸, R⁹ und R¹⁰ für Phenyl, das 1 oder 2 Cyanogruppen trägt, steht bzw. stehen, wobei mindestens einer der Reste R⁷, R⁸, R⁹ und R¹⁰ für Phenyl, das 1 oder 2 Cyanogruppen trägt, steht; 0, 1, 2 oder 3 der Reste R¹, R², R³, R⁴, R⁵, R⁶, R⁷, R⁸, R⁹ und R¹⁰ für Phenyl, das unsubstituiert ist oder 1, 2 oder 3 Reste R^{Ar} trägt, stehen und die übrigen Reste R¹, R², R³, R⁴, R⁵, R⁶, R⁷, R⁸, R⁹ und R¹⁰ für Wasserstoff stehen.

4. Verbindung der Formel (I) nach einem der vorhergehenden Ansprüche, die einer Verbindung der allgemeinen Formel (I-A) 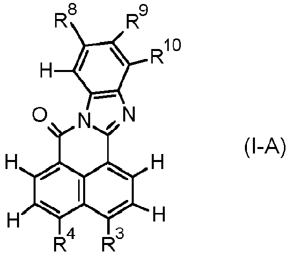 entspricht, worin
R³ und R⁴ jeweils unabhängig für Wasserstoff, Phenyl, das 1 oder 2 Cyanogruppen trägt, oder Phenyl, das unsubstituiert ist oder 1, 2 oder 3 Substituenten, die aus C₁-C₁₀-Alkyl ausgewählt sind, trägt, stehen und
mindestens einer der Reste R⁸, R⁹ und R¹⁰ für Phenyl, das 1 oder 2 Cyanogruppen trägt, steht und die übrigen Reste R⁸, R⁹ und R¹⁰ aus der Gruppe bestehend aus Wasserstoff und Phenyl, das unsubstituiert ist oder 1, 2 oder 3 Substituenten, die aus C₁-C₁₀-Alkyl ausgewählt sind, trägt, ausgewählt sind.

5. Verwendung einer Verbindung der Formel (I) gemäß einem der Ansprüche 1 bis 4 oder einer Mischung davon in Farbkonvertern zur Konvertierung von durch eine blaue LED mit einer Emissionsmittenwellenlänge zwischen 400 nm und 480 nm emittiertem Licht in Licht einer zweiten, längeren Wellenlänge oder zur Konvertierung von durch eine weiße LED mit einer korrelierten Farbtemperatur zwischen 3000 K und 20.000 K emittiertem Licht zur Bereitstellung von weißem Licht mit einer niedrigeren korrelierten Farbtemperatur.

6. Farbkonverter, umfassend in einer polymeren Matrix
- mindestens eine Naphthoylenbenzimidazol-Verbindung der Formel (I) gemäß einem der Ansprüche 1 bis 4 als Fluoreszenzfarbstoff und mindestens einen weiteren organischen Fluoreszenzfarbstoff, der aus
(a) einem organischen orangefarbenen Fluoreszenzfarbstoff mit einem Emissionsmaximum im Wellenlängenbereich von 540 bis weniger als 600 nm und Mischungen davon und
(b) einem organischen roten Fluoreszenzfarbstoff mit einem Emissionsmaximum im Wellenlängenbereich von 600 bis 670 nm und Mischungen davon
ausgewählt ist;
- und gegebenenfalls mindestens ein anorganisches Weißpigment als Streukörper.

7. Farbkonverter nach Anspruch 6, wobei der organische orangefarbene Fluoreszenzfarbstoff mit einem Emissionsmaximum im Wellenlängenbereich von 540 bis weniger als 600 nm aus
(a.1) einer kerncyanierten Naphthoylenbenzimidazol-Verbindung der Formel (II) 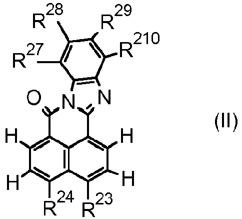 worin
eines von R²³ oder R²⁴ unabhängig voneinander für Cyano steht und der andere Rest R²³ oder R²⁴ aus Cyano, Phenyl, 4-Cyanophenyl und Phenyl, das 1, 2 oder 3 Substituenten, die aus C₁-C₁₀-Alkyl ausgewählt sind, trägt, ausgewählt ist;
R²⁷, R²⁸, R²⁹ und R²¹⁰ unabhängig voneinander für Wasserstoff, Phenyl, 4-Cyanophenyl oder Phenyl, das 1, 2 oder 3 Substituenten, die aus C₁-C₁₀-Alkyl ausgewählt sind, trägt;
und Mischungen davon;
(a.2) einer Perylenbisimid-Verbindung der Formel (III) 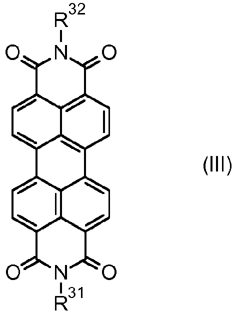 worin
R³¹ und R³² unabhängig voneinander für C₁-C₃₀-Alkyl, C₃-C₈-Cycloalkyl, Aryl, Heteroaryl oder Aryl-C₁-C₁₀-alkylen stehen, wobei der (hetero)aromatische Ring in den 3 letztgenannten Resten unsubstituiert oder ein- oder mehrfach durch C₁-C₁₀-Alkyl substituiert ist;
(a.3) einer Perylenbisimid-Verbindung mit starren 2,2'-Biphenoxybrücken der Formel (IV) 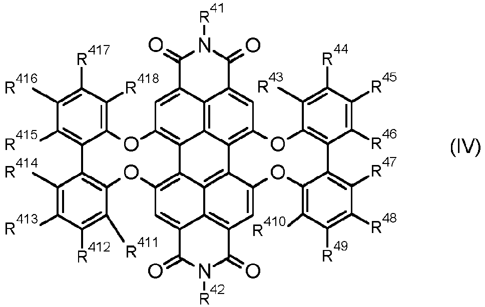 worin
R⁴¹ und R⁴² unabhängig voneinander aus Wasserstoff, C₁-C₁₀-Alkyl, das unsubstituiert oder durch C₆-C₁₀-Aryl, das wiederum unsubstituiert oder durch 1, 2 oder 3 C₁-C₁₀-Alkyl substituiert ist, substituiert ist, C₃-C₈-Cycloalkyl, das unsubstituiert oder durch 1, 2 oder 3 C₁-C₁₀-Alkyl substituiert ist, und C₆-C₁₀-Aryl, das unsubstituiert oder durch 1, 2 oder 3 C₁-C₁₀-Alkyl substituiert ist, ausgewählt ist;
R⁴³, R⁴⁴, R⁴⁵, R⁴⁶, R⁴⁷, R⁴⁸, R⁴⁹, R⁴¹⁰, R⁴¹¹,R⁴¹², R⁴¹³, R⁴¹⁴, R⁴¹⁵, R⁴¹⁶, R⁴¹⁷ und R⁴¹⁸ unabhängig voneinander aus Wasserstoff, Halogen, C₁-C₁₈-Alkyl, C₆-C₁₀-Aryl, C₆-C₁₀-Aryl-C₁-C₁₀-alkylen, C₆-C₁₀-Aryloxy und C₆-C₁₀-Arylthio ausgewählt sind, wobei die Arylgruppierung von C₆-C₁₀-Aryl, C₆-C₁₀-Aryl-C₁-C₁₀-alkylen, C₆-C₁₀-Aryloxy und C₆-C₁₀-Arylthio unsubstituiert oder durch ein oder mehrere C₁-C₁₀-Alkyl substituiert ist;
und Mischungen davon
ausgewählt ist.

8. Farbkonverter nach Anspruch 7, wobei der organische orangefarbene Fluoreszenzfarbstoff mit einem Emissionsmaximum im Wellenlängenbereich von 540 bis weniger als 600 nm aus Farbstoffen der Formeln (II.1), (III.1) und (IV.1) 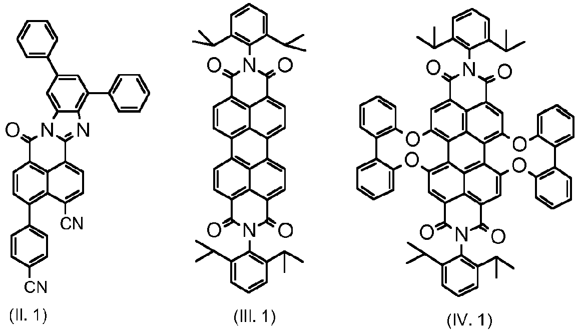 ausgewählt ist.

9. Farbkonverter nach einem der Ansprüche 6 bis 8, wobei es sich bei dem organischen roten Fluoreszenzfarbstoff mit einem Emissionsmaximum im Wellenlängenbereich von 600 bis 670 nm um eine Perylenbisimid-Verbindung der Formel (V) 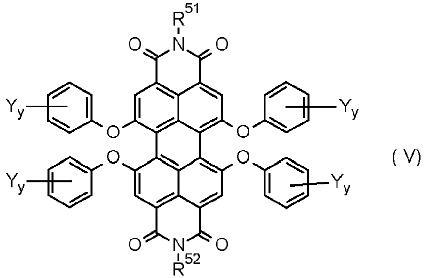 handelt, worin R
⁵¹ und R⁵² unabhängig voneinander für C₁-C₁₀-Alkyl, das unsubstituiert oder durch C₆-C₁₀-Aryl, das wiederum unsubstituiert oder durch 1, 2 oder 3 C₁-C₁₀-Alkyl substituiert ist, substituiert ist, C₃-C₈-Cycloalkyl, das unsubstituiert oder durch 1, 2 oder 3 C₁-C₁₀-Alkyl substituiert ist, oder C₆-C₁₀-Aryl, das unsubstituiert oder durch 1, 2 oder 3 C₁-C₁₀-Alkyl substituiert ist,
stehen;
Y unabhängig voneinander und unabhängig von jedem Vorkommen für C₁-C₁₀-Alkyl, Phenyl oder Phenyl, das durch 1, 2 oder 3 C₁-C₆-Alkyl substituiert ist, C₁-C₆-Alkoxy-C₁-C₆-alkyl und C₁-C₆-Alkoxy ausgewählt ist und
y unabhängig voneinander und unabhängig von jedem Vorkommen für 0, 1, 2 oder 3 steht.

10. Farbkonverter nach Anspruch 9, wobei der organische rote Fluoreszenzfarbstoff mit einem Emissionsmaximum im Wellenlängenbereich von 600 bis 670 nm aus einer Perylenbisimid-Verbindung der Formeln (V.1), (V.2) und (V.3) 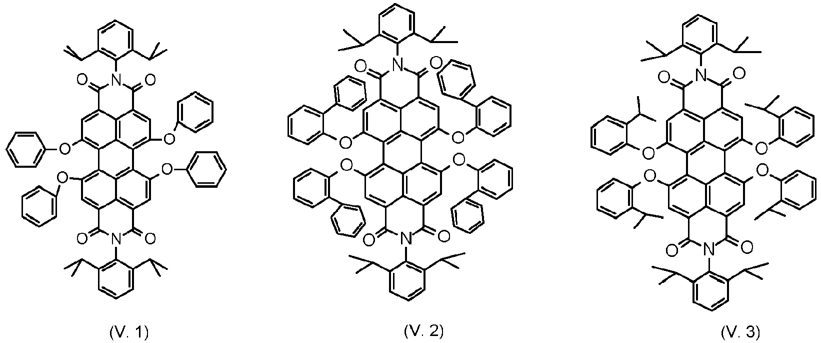 ausgewählt ist.

11. Farbkonverter nach einem der Ansprüche 6 bis 10, umfassend eine Kombination von organischen Fluoreszenzfarbstoffen, die aus einer Verbindung der Formeln (I-A) + (11.1) + (V.1), (I-A) + (11.1) + (V.2), (I-A) + (11.1) + (V.3), (I-A) + (III.1) + (V.1), (I-A) + (III.1) + (V.2), (I-A) + (III.1) + (V.3), (I-A) + (IV.1) + (V.1), (I-A) + (IV.1) + (V.2) und (I-A) + (IV.1) + (V.3) ausgewählt ist.

12. Farbkonverter nach einem der Ansprüche 6 bis 11, wobei die polymere Matrix im Wesentlichen aus Polystyrol, Polycarbonat, Polymethylmethacrylat, Polyvinylpyrrolidon, Polymethacrylat, Polyvinylacetat, Polyvinylchlorid, Polybuten, Silikon, Polyacrylat, Epoxidharz, Polyvinylalkohol, Poly-(ethylen-vinylalkohol)-Copolymer, Polyacrylnitril, Polyvinylidenchlorid, Poly(styrol-acrylnitril) (SAN), Polybutylenterephthalat, Polyethylenterephthalat, Polyvinylbutyrat, Polyvinylchlorid, Polyamiden, Polyoxymethylenen, Polyimiden, Polyetherimiden oder Mischungen davon, vorzugsweise Polystyrol, Polycarbonat oder Polyethylenterephthalat, besteht.

13. Verwendung eines Farbkonverters zur Konvertierung von von einer blauen LED mit einer Emissionsmittenwellenlänge zwischen 400 nm und 480 nm erzeugtem Licht zur Bereitstellung von weißem Licht oder zur Konvertierung von durch eine weiße LED mit einer korrelierten Farbtemperatur zwischen 3000 K und 20.000 K erzeugtem Licht zur Bereitstellung von weißem Licht mit einer niedrigeren korrelierten Farbtemperatur.

14. Beleuchtungsvorrichtung, umfassend
(i) mindestens eine LED, die aus einer blauen LED mit einer Emissionsmittenwellenlänge zwischen 400 nm und 480 nm und einer weißen LED mit einer korrelierten Farbtemperatur zwischen 3000 K und 20.000 K ausgewählt ist; und
(ii) mindestens einen Farbkonverter gemäß einem der Ansprüche 6 bis 12,
wobei sich der mindestens eine Farbkonverter in einer entfernten Anordnung von der mindestens einen LED befindet.

15. Vorrichtung, die bei Beleuchtung Strom erzeugt, mit einer Photovoltaikzelle und dem Farbkonverter gemäß einem der Ansprüche 6 bis 12, wobei mindestens ein Teil des nicht von der Photovoltaikzelle absorbierten Lichts von dem Farbkonverter absorbiert wird.

## Revendications

1. Composé de type naphtoylènebenzimidazole de formule (I) 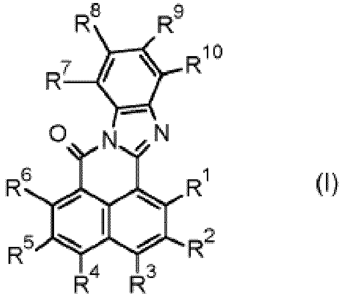 au moins l'un parmi les radicaux R¹, R², R³, R⁴, R⁵, R⁶, R⁷, R⁸, R⁹ et R¹⁰, indépendamment les uns des autres, étant aryle qui porte un, deux ou trois groupes cyano et 0, 1, 2, 3 ou 4 substituants R^{Ar} et les radicaux restants R¹, R², R³, R⁴, R⁵, R⁶, R⁷, R⁸, R⁹ et R¹⁰, indépendamment les uns des autres, étant choisis parmi hydrogène et aryle qui est non substitué ou porte 1, 2, 3, 4 ou 5 substituants R^{Ar}, et au moins l'un parmi les radicaux R⁷, R⁸, R⁹ et R¹⁰ étant aryle qui porte 1, 2 ou 3 groupes cyano et 0, 1, 2, 3 ou 4 substituants R^{Ar} et les radicaux restants R⁷, R⁸, R⁹ et R¹⁰ étant, indépendamment les uns des autres, choisis dans le groupe constitué par hydrogène et aryle qui est non substitué ou porte 1, 2 ou 3 substituants R^{Ar} ;
dans lesquels
R^{Ar}, indépendamment les uns des autres et indépendamment de chaque occurrence, est choisi parmi halogène, C₁₋₃₀-alkyle, C₂₋₃₀-alcényle, C₂₋₃₀-alcényle, dans lesquels les trois derniers radicaux sont non substitués ou portent un ou plusieurs groupes R^{a}, C₃₋₈-cycloalkyle, hétérocyclyle à 3 à 8 chaînons, dans lesquels les deux derniers radicaux sont non substitués ou portent un ou plusieurs groupes R^{b}, aryle et hétéroaryle, dans lesquels les deux derniers radicaux sont non substitués ou portent un ou plusieurs groupes R^{c}, dans lesquels
R^{a}, indépendamment les uns des autres et indépendamment de chaque occurrence, est choisi parmi cyano, halogène, C₃₋₈-cycloalkyle, hétérocyclyle à 3 à 8 chaînons, aryle et hétéroaryle, dans lesquels C₃₋₈-cycloalkyle, hétérocyclyle à 3 à 8 chaînons sont non substitués ou portent un ou plusieurs groupes R^{b1}, et dans lesquels aryle et hétéroaryle sont non substitués ou portent un ou plusieurs groupes R^{c1} ;
R^{b}, indépendamment les uns des autres et indépendamment chaque occurrence, est choisi parmi cyano, halogène, C₁₋₁₈-alkyle, C₃₋₈-cycloalkyle, hétérocyclyle à 3 à 8 chaînons, aryle et hétéroaryle, dans lesquels C₃₋₈-cycloalkyle, hétérocyclyle à 3 à 8 chaînons sont non substitués ou portent un ou plusieurs groupes R^{b1}, et dans lesquels aryle et hétéroaryle sont non substitués ou portent un ou plusieurs groupes R^{c1} ;
R^{c}, indépendamment les uns des autres et indépendamment de chaque occurrence, est choisi parmi cyano, halogène, C₁₋₁₈-alkyle, C₃₋₈-cycloalkyle, hétérocyclyle à 3 à 8 chaînons, aryle et hétéroaryle, dans lesquels C₃₋₈-cycloalkyle, hétérocyclyle à 3 à 8 chaînons sont non substitués ou portent un ou plusieurs groupes R^{b1}, et dans lesquels aryle et hétéroaryle sont non substitués ou portent un ou plusieurs groupes R^{c1} ;
R^{b1}, indépendamment les uns des autres et indépendamment de chaque occurrence, est choisi parmi halogène, C₁₋₁₈-alkyle et c₁₋₁₈-halogénoalkyle,
R^{c1}, indépendamment les uns des autres et indépendamment de chaque occurrence, est choisi parmi halogène, C₁₋₁₈-alkyle et C₁₋₁₈-halogénoalkyle ;
ou un mélange correspondant.

2. Composé de formule (I) selon la revendication 1, R^{Ar} étant C₁₋₁₀-alkyle, C₁₋₆-halogénoalkyle, C₃₋₈-cycloalkyle ou phényle qui est non substitué ou porte 1, 2 ou 3 radicaux R^{c}.

3. Composé de formule (I) selon la revendication 2, 1, 2, 3 ou 4 parmi les radicaux R¹, R², R³, R⁴, R⁵, R⁶, R⁷, R⁸, R⁹ et R¹⁰ étant phényle qui porte 1 ou 2 groupes cyano, au moins l'un parmi les radicaux R⁷, R⁸, R⁹ et R¹⁰ étant phényle qui porte 1 ou 2 groupes cyano ; 0, 1, 2 ou 3 parmi les radicaux R¹, R², R³, R⁴, R⁵, R⁶, R⁷, R⁸, R⁹ et R¹⁰ étant phényle qui est non substitué ou porte 1, 2 ou 3 radicaux R^{Ar} ; et les radicaux restants R¹, R², R³, R⁴, R⁵, R⁶, R⁷, R⁸, R⁹ et R¹⁰ étant hydrogène.

4. Composé de formule (I) selon l'une quelconque des revendications précédentes, qui correspond à un composé de formule (I-A) 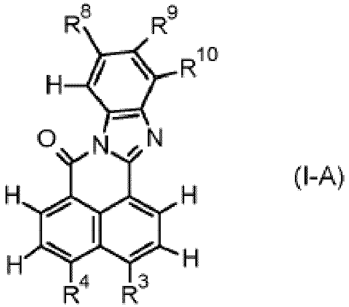
R³ et R⁴ étant chacun indépendamment hydrogène, phényle qui porte 1 ou 2 groupes cyano ou phényle qui est non substitué ou porte 1, 2 ou 3 substituants choisis parmi C₁₋₁₀-alkyle ; et
au moins l'un parmi les radicaux R⁸, R⁹ et R¹⁰ étant phényle qui porte 1 ou 2 groupes cyano ; et les radicaux restants R⁸, R⁹ et R¹⁰ étant choisis dans le groupe constitué par hydrogène et phényle qui est non substitué ou porte 1, 2 ou 3 substituants choisis parmi C₁₋₁₀-alkyle.

5. Utilisation d'un composé de formule (I) tel que défini dans l'une quelconque des revendications 1 à 4 ou d'un mélange correspondant dans des convertisseurs de couleur pour la conversion de lumière émise par une LED bleue dotée d'une longueur d'émission centrale comprise entre 400 nm et 480 nm en lumière d'une deuxième longueur d'onde plus longue ou pour la conversion de lumière émise par une LED blanche possédant une température de couleur corrélée comprise entre 3 000 K et 20 000 K pour fournir de la lumière blanche possédant une température de couleur corrélée inférieure.

6. Convertisseur de couleur comprenant dans une matrice polymérique
- au moins un composé de type naphtoylènebenzimidazole de formule (I) tel que défini dans l'une quelconque des revendications 1 à 4 en tant que colorant fluorescent et au moins un colorant fluorescent organique supplémentaire qui est choisi parmi
(a) un colorant fluorescent orange organique possédant un maximum d'émission dans la plage de longueurs d'onde de 540 à moins de 600 nm et des mélanges correspondants ; et
(b) un colorant fluorescent rouge organique possédant un maximum d'émission dans la plage de longueurs d'onde de 600 à 670 nm et des mélanges correspondants ; et
des mélanges correspondants ;
- et éventuellement au moins un pigment blanc inorganique en tant qu'un corps de diffusion.

7. Convertisseur de couleur selon la revendication 6, le colorant fluorescent orange organique possédant un maximum d'émission dans la plage de longueurs d'onde de 540 à moins de 600 nm étant choisi parmi
(a.1) un composé de type naphtoylènebenzimidazole à noyau cyanaté de formule (II) 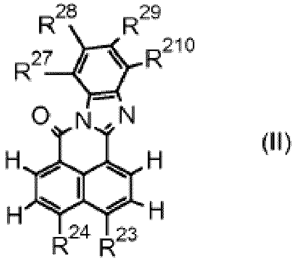
l'un parmi R²³ et R²⁴, indépendamment l'un de l'autre, étant cyano et l'autre radical R²³ ou R²⁴ étant choisi parmi cyano, phényle, 4-cyanophényle et phényle qui porte 1, 2 ou 3 substituants choisis parmi C₁₋₁₀-alkyle ;
R²⁷, R²⁸, R²⁹ et R²¹⁰, indépendamment les uns des autres, étant hydrogène, phényle, 4-cyanophényle ou phényle qui porte 1, 2 ou 3 substituants choisis parmi C₁₋₁₀-alkyle,
et des mélanges correspondants,
(a.2) un composé de type bisimide de pérylène de formule (III) 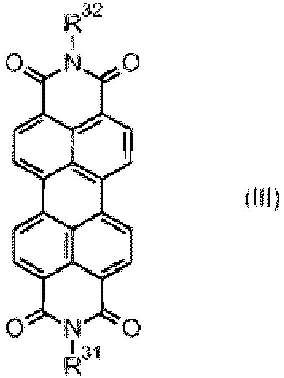 R³¹ et R³², indépendamment l'un de l'autre, étant C₁₋₃₀-alkyle, C₃₋₈-cycloalkyle, aryle, hétéroaryle ou aryl-C₁₋₁₀-alkylène, dans lesquels le cycle (hétéro)aromatique dans les trois derniers radicaux est non substitué ou monosubstitué ou polysubstitué par C₁₋₁₀-alkyle ;
(a.3) un composé de bisimide de pérylène doté de ponts rigides de type 2,2'-biphénoxy de formule (IV) 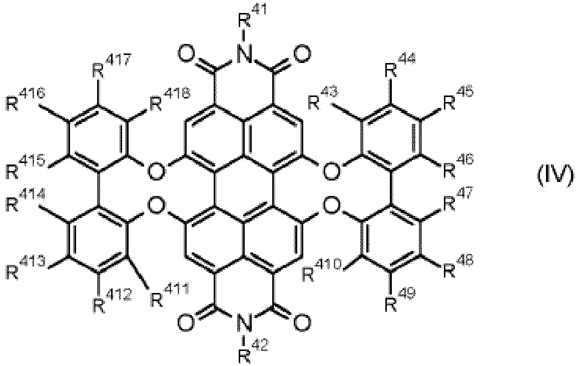
R⁴¹ et R⁴², indépendamment l'un de l'autre, étant choisis parmi hydrogène, C₁₋₁₀-alkyle, qui est non substitué ou substitué par C₆₋₁₀-aryle qui est à son tour non substitué ou substitué par 1, 2 ou 3 C₁₋₁₀-alkyle, C₃₋₈-cycloalkyle, qui est non substitué ou substitué par 1, 2 ou 3 C₁₋₁₀-alkyle, et C₆₋₁₀-aryle qui est non substitué ou substitué par 1, 2 ou 3 C₁₋₁₀-alkyle ;
R⁴³, R⁴⁴, R⁴⁵, R⁴⁶, R⁴⁷, R⁴⁸, R⁴⁹, R⁴¹⁰, R⁴¹¹, R⁴¹²,R⁴¹³, R⁴¹⁴, R⁴¹⁵, R⁴¹⁶, R⁴¹⁷ et R⁴¹⁸, indépendamment les uns des autres, étant choisi parmi hydrogène, halogène, C₁₋₁₈-alkyle, C₆₋₁₀-aryle, C₆₋₁₀-aryl-C₁₋₁₀-alkylène, C₆₋₁₀- aryloxy et C₆₋₁₀-arylthio, dans lesquels le fragment aryle de C₆₋₁₀-aryle, C₆₋₁₀-aryl-C₁₋₁₀-alkylène, C₆₋₁₀-aryloxy et C₆₋₁₀-arylthio est non substitué ou substitué par un ou plusieurs C₁₋₁₀-alkyle ;
et des mélanges correspondants.

8. Convertisseur de couleur selon la revendication 7, le colorant fluorescent orange organique possédant un maximum d'émission dans la plage de longueurs d'onde de 540 à moins de 600 nm étant choisi parmi des colorants des formules (II.1), (III.1) et (IV.1)

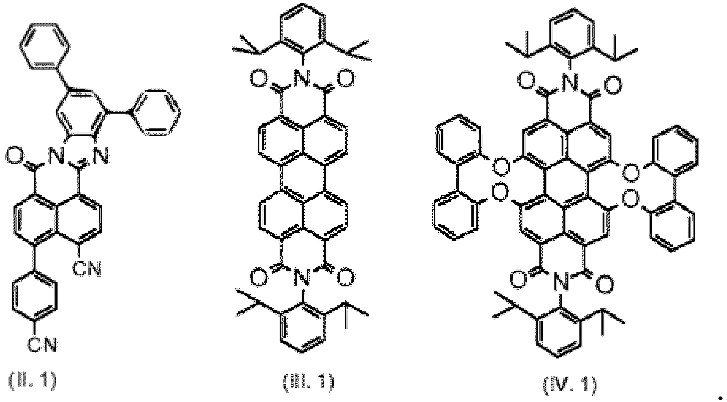

9. Convertisseur de couleur selon l'une quelconque des revendications 6 à 8, le colorant fluorescent rouge organique possédant un maximum d'émission dans la plage de longueurs d'onde de 600 à 670 nm étant un composé de type bisimide de pérylène de formule (V) 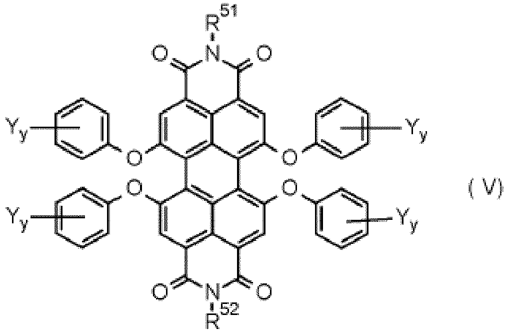
R⁵¹ et R⁵², indépendamment l'un de l'autre, étant C₁-₁₀-alkyle, qui est non substitué ou substitué par C₆₋₁₀-aryle qui est à son tour non substitué ou substitué par 1, 2 ou 3 C₁₋₁₀-alkyle, C₃₋₈-cycloalkyle, qui est non substitué ou substitué par 1, 2 ou 3 C₁₋₁₀-alkyle, ou C₆₋₁₀-aryle qui est non substitué ou substitué par 1, 2 ou 3 C₁₋₁₀-alkyle ;
Y, indépendamment les uns des autres et indépendamment de chaque occurrence, étant C₁₋₁₀-alkyle, phényle ou phényle qui est substitué par 1, 2 ou 3 C₁₋₆-alkyle, C₁₋₆-alcoxy-C₁₋₆-alkyle et C₁₋₆-alcoxy ; et
y, indépendamment les uns des autres et indépendamment de chaque occurrence, étant 0, 1, 2, ou 3.

10. Convertisseur de couleur selon la revendication 9, le colorant fluorescent rouge organique possédant un maximum d'émission dans la plage de longueurs d'onde de 600 à 670 nm étant choisi parmi un composé de type bisimide de pérylène parmi les formules (V.1), (V.2), et (V.3)

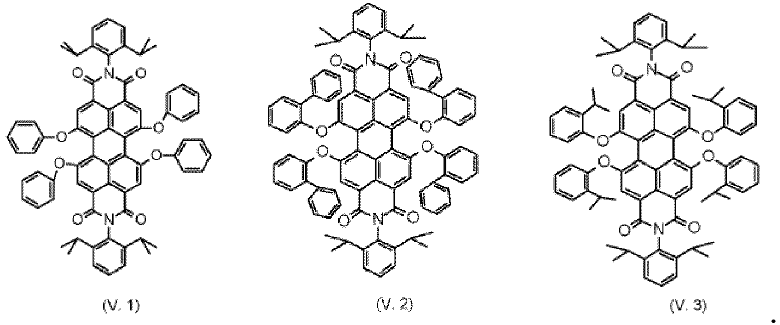

11. Convertisseur de couleur selon l'une quelconque des revendications 6 à 10, comprenant une combinaison de colorants fluorescents organiques choisie parmi un composé parmi les formules (I-A) + (II.1) + (V.1), (I-A) + (II.1) + (V.2), (I-A) + (II.1) + (V.3), (I-A) + (III.1) + (V.1), (I-A) + (III.1) + (V.2), (I-A) + (III.1) + (V.3), (I-A) + (IV.1) + (V.1), (I-A) + (IV.1) + (V.2), et (I-A) + (IV.1) + (V.3).

12. Convertisseur de couleur selon l'une quelconque des revendications 6 à 11, la matrice polymérique étant essentiellement constituée de polystyrène, de polycarbonate, de poly(méthacrylate de méthyle), de polyvinylpyrrolidone, de polyméthacrylate, de poly(acétate de vinyle), de poly(chlorure de vinyle), de polybutène, de silicone, de polyacrylate, des résine époxy, de poly(alcool vinylique), d'un copolymère de poly(éthylène-alcool vinylique), de polyacrylonitrile, de poly(chlorure de vinylidène), de polystyrène acrylonitrile (SAN), de poly(téréphtalate de butylène), de poly(téréphtalate d'éthylène), de poly(butyrate de vinyle), de poly(chlorure de vinyle), de polyamides, de polyoxyméthylènes, de polyimides, de polyétherimides ou des mélanges correspondants, préférablement de polystyrène, de polycarbonate, ou de poly(téréphtalate d'éthylène).

13. Utilisation d'un convertisseur de couleur tel que défini dans l'une quelconque des revendications 6 à 12 pour la conversion de lumière générée par une LED bleue dotée d'une longueur d'onde d'émission centrale comprise entre 400 nm et 480 nm pour fournir de la lumière blanche ou pour la conversion de lumière générée par une LED blanche possédant une température de couleur corrélée comprise entre 3 000 K et 20 000 K pour fournir de la lumière blanche possédant une température de couleur corrélée inférieure.

14. Dispositif d'éclairage comprenant
(i) au moins une LED choisie parmi une LED bleue dotée d'une longueur d'onde d'émission centrale comprise entre 400 nm et 480 nm et une LED blanche possédant une température de couleur corrélée comprise entre 3 000 K et 20 000 K ; et
(ii) au moins un convertisseur de couleur tel que défini dans l'une quelconque des revendications 6 à 12,
l'au moins un convertisseur de couleur se trouvant dans un agencement à distance de l'au moins une LED.

15. Dispositif de production de puissance électrique par illumination comprenant une cellule photovoltaïque et le convertisseur de couleur tel que défini dans l'une quelconque des revendications 6 à 12, dans lequel au moins une partie de la lumière non absorbée par la cellule photovoltaïque est absorbée par le convertisseur de couleur.
